# EUROPEAN PATENT APPLICATION

(11) **EP 2 810 962 A1**
(43) Date of publication of application: **10.12.2014**
(21) Application number: 13743801.6
(22) Date of filing: 29.01.2013
(51) Int. Cl.: C08F 20/38, C07C 251/64, C07C 323/47, C08F 8/26, C08F 20/36, C08J 7/00, C09D 4/02, C09K 3/18

(54) **COMPOUND, POLYMER, CURABLE COMPOSITION, COATING COMPOSITION, ARTICLE HAVING CURED FILM, ARTICLE HAVING PATTERN OF LYOPHILIC REGIONS AND LYOPHOBIC REGIONS, AND METHOD FOR PRODUCING SAME**

(30) Priority: 31.01.2012 JP 2012018086; 26.03.2012 JP 2012069928
(71) Applicant: Asahi Glass Company, Limited, Tokyo 100-8405 (JP)
(72) Inventor: ITO, Masahiro, Tokyo 100-8405 (JP); TSURUOKA, Kaori, Tokyo 100-8405 (JP); KUWANA, Yasuhiro, Tokyo 100-8405 (JP); NAGAI, Yusuke, Tokyo 100-8405 (JP); FUKUDA, Miyako, Tokyo 100-8405 (JP)
(74) Representative: Müller-Boré & Partner Patentanwälte PartG mbB
(86) International application number: PCT/JP2013/051928
(87) International publication number: WO 2013/115195

(57) **Abstract**

To provide a curable composition which has favorable insulating property and liquid repellency, and from which a cured film having liquid repellency capable of being sufficiently converted to be liquid-philic even by irradiation with ultraviolet light having a wavelength of at least 300 nm, can be formed; an article having a cured film obtained by curing the curable composition and its production process.

A cured film is formed by using a curable composition comprising a polymer having units (u1) based on a compound represented by the following formula (m1): 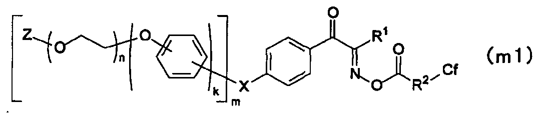 wherein R¹= a hydrogen atom, a methyl group or the like; R²= a bivalent organic group or the like; Cf= a C₁₋₂₀ fluoroalkyl group or the like, X=O, S, N or NH; m= 1 to 2; n= 0 to 4; k= 0 to 1; and Z= a (meth)acryloyl group or the like.

## Description

### TECHNICAL FIELD

The present invention relates to a compound having liquid repellency and capable of being converted to be liquid-philic by irradiation with ultraviolet light, a liquid repellent polymer, a curable composition, a coating composition, an article having a cured film, an article having a pattern of a liquid-philic region and a liquid repellent region, and a process for producing it.

### BACKGROUND ART

In the field of semiconductor devices, displays, light-emitting devices, etc., various functional thin films are practically used. A functional thin film is patterned by disposing a material having desired properties to a desired position. For example, for a thin-film transistor, a process for producing an electrode has been proposed by which a cured film having liquid repellency is formed on the surface of a substrate, the surface of the cured film is partially irradiated with ultraviolet light to convert the portion irradiated with ultraviolet light to be liquid-philic, and a composition for an electrode is selectively deposited to the portion of the cured film converted to be liquid-philic to form an electrode. This method attracts attention as a method to form an electrode having a desired pattern easily with a small number of steps as compared with a method by photolithography.

As a material for forming a liquid repellent cured film which can be converted to be liquid-philic by irradiation with ultraviolet light, for example, the following materials have been proposed.
(1) A material containing a photocatalyst (such as titanium dioxide) and a binder (such as organopolysiloxane) (Patent Document 1).
(2) A composition containing a low molecular weight fluorinated compound which can be decomposed and removed by irradiation with ultraviolet light (Patent Document 2).
(3) A polyimide having hydrophobic groups in its side chains (Patent Document 3).
(4) A polyimide having a thiolester bond in its main chain (Patent Document 4).

### PRIOR ART DOCUMENTS

### PATENT DOCUMENTS

- Patent Document 1:: Japanese Patent No. 4300012
- Patent Document 2:: WO2005/054256
- Patent Document 3:: JP-A-2005-310962
- Patent Document 4:: WO2008/133300

### DISCLOSURE OF INVENTION

### TECHNICAL PROBLEM

In a case where a liquid repellent cured film is formed by using the material (1), the photocatalyst remains in the cured film. In a case where an article having a functional thin film is a thin-film transistor, a semiconductor device or the like, the cured film functions as an insulation film, and the photocatalyst remaining in the insulation film adversely effects the properties (such as the insulating property) of the insulation film.

In a case where a liquid repellent cured film is formed by using the composition (2) containing a fluorinated compound, since the fluorinated compound has a relatively low molecular weight, the fluorinated compound is uniformly dispersed in the cured film, and the fluorinated compound will not be unevenly present on the surface of the cured film (the opposite side from the substrate). Accordingly, the liquid repellency of the surface of the cured film may sometimes be insufficient. Further, the obtained cured film may sometimes have insufficient insulating properties.

In a case where a liquid repellent cured film is formed by using the polyimide (3) or (4), the polyimide absorbs ultraviolet light at a relatively long wavelength side (for example, ultraviolet light having a wavelength of at least 300 nm). Accordingly, in a case where a light source commonly used as a light source for ultraviolet light such as a high pressure mercury lamp (i-line: 365 nm) or a YAG laser (third harmonic: 355 nm) is used, the sensitivity tends to be insufficient. Further, in recent years, a material having a dielectric constant lower than that of the polyimide is required in some cases.

The object of the present invention is to provide a compound and a polymer, which have favorable liquid repellency, which are decomposed in their molecule by irradiation with ultraviolet light having a wavelength of at least 300 nm, and from which a decomposition residue containing a fluoroalkyl group can leave.

Another object of the present invention is to provide a curable composition and a coating composition, from which a liquid repellent cured film having favorable insulating property and liquid repellency and capable of being sufficiently converted to be liquid-philic by irradiation with ultraviolet light having a wavelength of at least 300 nm, can be formed.

Another object of the present invention is to provide an article having a cured film having favorable insulating property and liquid repellency.

Still another object of the present invention is to provide an article having favorable insulating property and having a pattern of a liquid-philic region and a liquid repellent region, and its production process.

### SOLUTION TO PROBLEM

The present invention provides a compound, a polymer, a curable composition, a coating composition, an article having a cured film, an article having a pattern of a liquid-philic region and a liquid repellent region, and its production process according to the following [1] to [15].
[1] A compound represented by the following formula (m1): 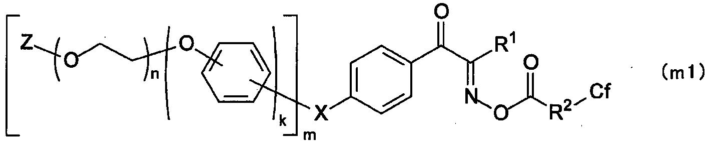 wherein R¹ is a hydrogen atom, a C₁₋₆ alkyl group or a phenyl group,
   R² is a single bond or a bivalent organic group having no fluorine atoms,
   Cf is a C₁₋₂₀ fluoroalkyl group or a C₂₋₂₀ fluoroalkyl group having an etheric oxygen atom between carbon atoms,
   X is an oxygen atom, a sulfur atom, a nitrogen atom or NH,
   m is 1 when X is an oxygen atom, a sulfur atom or NH, or 2 when X is a nitrogen atom,
   n is an integer of from 0 to 4,
   k is 0 or 1,
   Z is R⁴R⁵C=CR³-CO-, and
   each of R³, R⁴ and R⁵ which are independent of one another, is a hydrogen atom or a methyl group.
[2] The compound according to the above [1], wherein R² is a single bond, -(CH₂)_{w0}-(wherein w0 is an integer of from 1 to 6), -C₆H₄-, -C₆H₄O(CH₂)_{w1}- (wherein w1 in an integer of from 0 to 10), -C₆H₄COO(CH₂)_{w2}- (wherein w2 is an integer of from 0 to 10), -(CH₂)_{w3}COO(CH₂)_{w4}- (wherein w3 is an integer of from 1 to 10, and w4 is an integer of from 0 to 10), -CH₂O(CH₂)_{w5}- (wherein w5 is an integer of from 0 to 10) or -CH (CH₃)O(CH₂)_{w6}- (wherein w6 is an integer of from 0 to 10).
[3] A polymer having units (u1) based on the compound as defined in the above [1] or [2].
[4] The polymer according to the above [3], which further has units (u2) having a crosslinkable functional group and having no Cf group.
[5] A curable composition comprising the polymer as defined in the above [3] or [4].
[6] The curable composition according to the above [5], which further contains a radical polymerization initiator (D).
[7] The curable composition according to the above [5] or [6], which further contains a fluorinated polyarylene prepolymer (A) having a crosslinkable functional group.
[8] The curable composition according to the above [7], which further contains a compound (B) having a number average molecular weight of from 140 to 5,000, having a crosslinkable functional group and having no fluorine atoms.
[9] The curable composition according to any one of the above [5] to [8], wherein the fluorinated polyarylene prepolymer (A) is a prepolymer having a crosslinkable functional group and an ether bond, obtained by subjecting either one or both of a compound (x1) having a crosslinkable functional group and a phenolic hydroxy group and a compound (x2) having a crosslinkable functional group and a fluorinated aromatic ring, a compound (y) represented by the following formula (y), and a compound (z) having at least three phenolic hydroxy groups, to a condensation reaction in the presence of a hydrogen halide-removing agent: 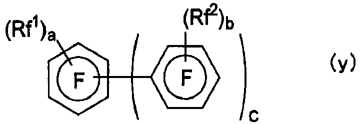 wherein c is an integer of from 0 to 3, a is an integer of from 0 to 3, b is an integer of from 0 to 3, Rf¹ is a fluoroalkyl group having at most 8 carbon atoms, provided that when there are a plurality of Rf¹'s, the plurality of Rf¹'s may be the same or different, Rf² is a fluoroalkyl group having at most 8 carbon atoms, provided that when there are a plurality of Rf²'s, the plurality of Rf²'s may be the same or different, and F in the aromatic ring represents that hydrogen atoms of the aromatic ring are all substituted by fluorine atoms.
[10] A coating composition comprising the curable composition as defined in any one of the above [5] to [9] and a solvent (E).
[11] An article comprising a substrate, and a cured film obtained by curing the curable composition as defined in any one of the above [5] to [9] on the surface of the substrate.
[12] An article having a pattern of a liquid-philic region and a liquid repellent region on the surface of a cured film, wherein the liquid repellent region comprises a cured film obtained by curing the curable composition as defined in any one of the above [5] to [9].
[13] The article according to the above [12], which further has at least one member selected from the group consisting of an electrode, a semiconductor layer, a conductor layer, a transistor material and a resin layer, formed on the surface of the liquid-philic region.
[14] A process for producing an article having a pattern of a liquid-philic region and a liquid repellent region, which comprises forming a film of the curable composition as defined in any one of the above [5] to [9] on the surface of a substrate, curing the film of the curable composition to form a cured film, and partially irradiating the surface of the cured film with ultraviolet light to form a pattern of a liquid-philic region and a liquid repellent region on the surface of the cured film.
[15] A process for producing an article having a pattern of a liquid-philic region and a liquid repellent region, which comprises forming a film of the curable composition as defined in any one of the above [5] to [9] on the surface of a substrate, partially irradiating the surface of the film of the curable composition with ultraviolet light to form a pattern of a liquid-philic region and a liquid repellent region on the surface of the film, and curing the curable composition.

### ADVANTAGEOUS EFFECTS OF INVENTION

According to the present invention, it is possible to provide a compound and a polymer, which have favorable liquid repellency, which are decomposed in their molecule by irradiation with ultraviolet light having a wavelength of at least 300 nm, and from which a decomposition residue containing a fluoroalkyl group can leave. According to the present invention, a curable composition and a coating composition, from which a liquid repellent cured film having favorable insulating property and liquid repellency, sufficiently converted to be liquid-philic by irradiation with ultraviolet light having a wavelength of at least 300 nm, can be formed.

According to the present invention, it is possible to provide an article having a cured film having favorable insulating property and liquid repellency.

According to the present invention, an article having favorable insulating property and having a pattern of a liquid-philic region and a liquid repellent region, and a process for efficiently producing the article.

### BRIEF DESCRIPTION OF DRAWINGS

Fig. 1 is a cross-sectional view illustrating an example of an organic thin-film transistor.
Fig. 2 is a cross-sectional view illustrating an example of a step for producing an organic thin-film transistor.
Fig. 3 is a cross-sectional view illustrating an example of a step for producing an organic thin-film transistor.

### DESCRIPTION OF EMBODIMENTS

In this specification, a compound represented by a formula (m1) will be referred to as a compound (m1). The same applies to compounds represented by other formulae. Further, a polymer having units (u1) based on the compound (m1) will sometimes be referred to as "a polymer (C)". A fluorinated polyarylene prepolymer having a crosslinkable functional group will sometimes be referred to as "a prepolymer (A)", and a compound (B) having a crosslinkable functional group and having no fluorine atoms will sometimes be referred to as "a compound (B)".

In this specification, "liquid repellency" generally means water repellency and oil repellency.

In this specification, "liquid-philicity" generally means hydrophilicity and lipophilicity.

In this specification, "converted to be liquid-philic" means that liquid-repellency is relatively changed to liquid-philicity, and specifically, the contact angle to water or an organic solvent is decreased.

In this specification, "fluoroalkyl group" means an alkyl group in which some or all of the hydrogen atoms are substituted by fluorine atoms, and "perfluoroalkyl group" is an alkyl group in which all the hydrogen atoms are substituted by fluorine atoms.

In this specification, "methacryloyl(oxy) group" generally means a methacryloyl group and a methacryloyloxy group. The same applies to "acryloyl(oxy) group".

In this specification, "(meth)acryloyl group" generally means an acryloyl group and a methacryloyl group. The same applies to "(meth)acryloyloxy group".

In this specification, "unit" is a repeating unit derived from a monomer, formed by polymerization of the monomer. The unit may be a unit directly formed by polymerization, or may be a unit having a part of the unit converted to another structure by treatment of the polymer.

In this specification, "monomer" means a compound having a functional group polymerizable by radicals.

In this specification, "crosslinkable functional group" means a functional group polymerizable by radicals.

The crosslinkable functional group may, for example, be a carbon-carbon unsaturated double bond which is polymerizable by radicals, a carbon-carbon unsaturated triple bond which is polymerizable by radicals, a ring which is to be opened by radicals, and groups containing them. The unsaturated double bond and the unsaturated triple bond may be present at the inside of a molecular chain (hereinafter, also referred to as "inside olefin type") or present at a terminal thereof (hereinafter, also referred to as "terminal olefin type"), but a terminal olefin type is preferred since its reactivity is high. The inside olefin type includes a case where an unsaturated double bond is present in a part of an aliphatic ring, such as cycloolefin. The terminal olefin type crosslinkable functional group is preferably an alkenyl group having at most 4 carbon atoms or an alkynyl group having at most 4 carbon atoms.

Specifically, the crosslinkable functional group may be a vinyl group, an allyl group, an isopropenyl group, a 3-butenyl group, a methacryloyl group, a methacryloyloxy group, an acryloyl group, an acryloyloxy group, a vinyloxy group, an allyloxy group, a trifluorovinyl group, a trifluorovinyloxy group, an ethynyl group, a 1-oxocyclopenta-2,5-dien-3-yl group or an oxirane ring. The crosslinkable functional group is preferably at least one member selected from the group consisting of a vinyl group, an allyl group, an ethynyl group, a vinyloxy group, an allyloxy group, an acryloyl group, an acryloyloxy group, a methacryloyl group and a methacryloyloxy group, since the reactivity is high, and a cured film having high crosslinking density can easily be obtained.

In this specification, the number average molecular weight (Mn) is a molecular weight calculated as polystyrene obtainable by measurement by gel permeation chromatography employing a calibration curve prepared by using a standard polystyrene sample having a known molecular weight.

### [Compound represented by the formula (m1)]

The compound of the present invention is a compound represented by the formula (m1), i.e. the compound (m1). The boundary between R² and Cf in the formula (m1) is defined so that the number of carbon atoms of Cf is the smallest. In other words, in a case where R² is not a single bond, R² is an organic group defined to have the largest number of carbon atoms under conditions that it is a bivalent organic group having no fluorine atoms.

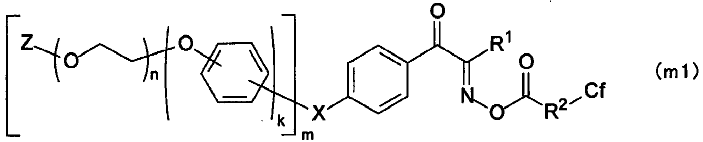

As the compound (m1), cis-trans isomers are present due to a double bond of an oxime. The compound (m1) in the present invention is not limited to one represented by the above formula, and the compound (m1) may consists of solely of a cis-form or a trans-form, or may be a mixture of them.

R¹ is a hydrogen atoms, a C₁₋₆ alkyl group or a phenyl group, and is preferably a C₁₋₆ alkyl group in view of favorable solubility of the compound (m1).

R² is a single bond or a bivalent organic group having no fluorine atoms. The bivalent organic group as R² may be -(CH₂)_{w0}- (wherein w0 is an integer of from 1 to 6), -C₆H₄-, -C₆H₄O(CH₂)_{w1}- (wherein w1 is an integer of from 0 to 10), -C₆H₄COO(CH₂)_{w2}-(wherein w2 is an integer of from 0 to 10), -(CH₂)_{w3}COO(CH₂)_{w4}- (wherein w3 is an integer of from 1 to 10, and w4 is an integer of from 0 to 10), -CH₂O(CH₂)_{w5}- (wherein w5 is an integer of from 0 to 10) or -CH(CH₃)O(CH₂)_{w6}- (wherein w6 is an integer of from 0 to 10). Preferred is -C₆H₄-, -C₆H₄O(CH₂)_{w1}- or -C₆H₄COO(CH₂)_{w2}-, whereby ultraviolet light having a wavelength of at least 300 nm is likely to be absorbed. Further, preferred is a single bond, -(CH₂)_{w0}-, -(CH₂)_{w3}COO(CH₂)_{w4}-, -CH₂O(CH₂)_{w5}- or - CH(CH₃)O(CH₂)_{w6}-, whereby the decomposition residue containing a Cf group is likely to be removed. Further, w0 is preferably an integer of from 1 to 3, w1 is preferably an integer of from 0 to 4, and is particularly preferably an integer of from 0 to 2, in view of easiness of preparation. w2 is preferably an integer of from 0 to 4, particularly preferably an integer of from 0 to 2, in view of easiness of preparation. w3 is preferably an integer of from 0 to 6, particularly preferably an integer of from 1 to 3, in view of easiness of preparation. w4 is preferably an integer of from 0 to 4, particularly preferably an integer of from 0 to 2 in view of easiness of preparation. w5 is preferably an integer of from 0 to 4, particularly preferably an integer of from 0 to 2 in view of easiness of preparation. w6 is preferably an integer of from 0 to 4, particularly preferably an integer of from 0 to 2 in view of easiness of preparation.

Cf is a C₁₋₂₀ fluoroalkyl group or a C₂₋₂₀ fluoroalkyl group having an etheric oxygen atom between carbon atoms. Cf may be linear or branched, and to its carbon atom, a halogen atom other than fluorine, such as a chlorine atom, may be bonded in addition to hydrogen atoms.

In Cf, the terminal group bonded to R² may, for example, be a difluoromethylene group, a fluoromethylene group, a bis(trifluoromethyl)methylene group, a fluoro(trifluoromethyl)methylene group, a (trifluoromethyl)methylene group, a methyl (trifluoromethyl)methylene group or a fluoro(methyl)methylene group. In these examples, the trifluoromethyl group is an example of a side chain group having fluorine atoms, and the methyl group is an example of a side chain group having no fluorine atoms.

The number of carbon atoms in the Cf group is preferably from 2 to 20, more preferably from 2 to 15, particularly preferably from 4 to 8, in view of excellent liquid repellency and favorable compatibility with other monomers. Further, the number of carbon atoms in the Cf group is preferably at most 6, more preferably from 2 to 6, particularly preferably from 4 to 6 in view of low environmental burden.

The Cf group has a proportion of the number of fluorine atoms of at least 80% to the total number of fluorine atoms, hydrogen atoms and halogen atoms other than the fluorine atoms, in view of more favorable liquid repellency of the surface of a cured film, particularly preferably 100%, that is, the Cf group is particularly preferably a C₁₋₂₀ perfluoroalkyl group or a C₂₋₂₀ perfluoroalkyl group having an etheric oxygen atom between carbon atoms.

The Cf group may be linear or branched.

Specifically, the Cf group may be -CF₃, -CF₂CF₃, -CF(CF₃)₂, -CH(CF₃)₂, -CF₂CHF₂, -(CF₂)₂CF₃, -(CF₂)₃CF₃, -(CF₂)₄CF₃, -(CF₂)₅CF₃, -(CF₂)₆CF₃, -(CF₂)₇CF₃, -(CF₂)₈CF₃, -(CF₂)₉CF₃, -(CF₂)₁₁CF₃, -(CF₂)₁₅CF₃, -CF(CF₃)O(CF₂)₅CF₃, -CF₂OCF₂CF₂OCF₂CF₃, -CF₂O(CF₂CF₂O)ₚCF₃ (wherein p is an integer of from 1 to 8), -CF(CF₃)O(CF₂CF(CF₃)O)_{q}C₆F₁₃ (wherein q is an integer of from 1 to 4), or -CF(CF₃)O(CF₂CF(CF₃)O)ᵣC₃F₇ (wherein r is an integer of from 0 to 5).

X is an oxygen atom, a sulfur atom, a nitrogen atom or NH, preferably an oxygen atom, a sulfur atom or NH, whereby a polymer (C) is easily produced (the polymer (C) is less likely to be gelated), and is more preferably an oxygen atom or a sulfur atom in view of availability of the raw material, and is particularly preferably a sulfur atom in view of easiness of preparation of the compound (m1).

m is 1 when X is an oxygen atom, a sulfur atom or NH, or 2 when X is a nitrogen atom, and is preferably 1, whereby the polymer (C) is easily produced (the polymer (C) is less likely to be gelated).

n is an integer of from 0 to 4, and in view of availability of the raw material and easiness of preparation, preferably an integer of from 0 to 2, particularly preferably an integer of from 0 to 1.

k is 0 or 1, and is particularly preferably 1 in view of availability of the raw material and easiness of preparation. The positional relation of X and O in -O-Ph-X- (wherein Ph is a phenylene group) is preferably a para-position in view of availability of the raw material.

Z is R⁴R⁵C=CR³-CO-.

Each of R³, R⁴ and R⁵ which are independent of one another, is a hydrogen atom or a methyl group, and in view of high reactivity, it is preferred that R³ is a hydrogen atom or a methyl group and each of R⁴ and R⁵ is a hydrogen atom. That is, Z is preferably an acryloyl group or a methacryloyl group.

### (Method for producing compound (m1))

To produce the compound (m1), a method to carry out reaction represented by the following formula may be mentioned. HO- of a compound (a1) is esterified with a compound (b1) in the presence of a tertiary amine to obtain a compound (c1), which is converted to an oxime by a nitrite to obtain a compound (d1). Then, -OH of the compound (d1) is esterified with a compound (e1) in the presence of a carbodiimide to obtain a compound (m1):

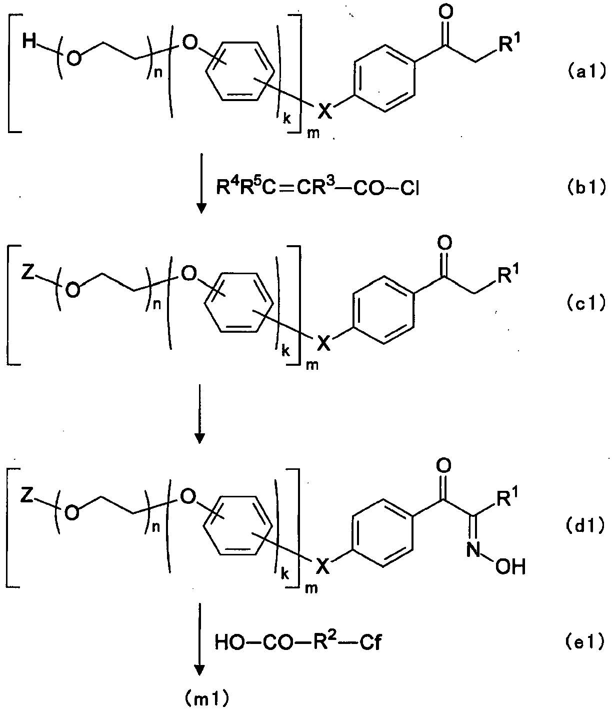

The compound (a1) may be prepared by a known production method. For example, in a case where X is a sulfur atom, n is 0 and k is 1, it may be prepared by reacting HO-C₆H₄-SH with Br-C₆H₄-CO-CH₂-R¹.

### [Polymer (C)]

The polymer (C) which is the polymer of the present invention is a polymer having units (hereinafter sometimes referred to as "units (u1)" based on the compound (m1) of the present invention.

The polymer (C) preferably further has units (hereinafter sometimes referred to as "units (u2)") having a crosslinkable functional group and having no Cf group in view of the hardness, the solvent resistance, etc. of a water repellent film.

The polymer (C) may have units (hereinafter sometimes referred to as units (u3)" other than the units (u1) and the units (u2).

The units (u1), the units (u2) and the units (u3) in the polymer (C) may be bonded randomly or may be bonded in a block.

The fluorine content of the polymer (C) is preferably from 5 to 60 weight%, particularly preferably from 8 to 40 weight%. When the fluorine content is at least the lower limit value of the above range, the liquid repellency of the surface of a cured film will be more favorable. When it is at most the upper limit value of the above range, the adhesion between the cured film and a layer adjacent thereto will be favorable.

The number average molecular weight (Mn) of the polymer (C) is preferably from 1,000 to 50,000, particularly preferably from 3,000 to 20,000. When the number average molecular weight (Mn) is at least the lower limit value of the above range, the polymer (C) will sufficiently moves to the surface of the cured film, whereby more favorable liquid repellency will be obtained. When it is at most the upper limit value of the above range, compatibility with the prepolymer (A) in the curable composition will be favorable, and a cured film without defects will be formed.

### (Unit (u1))

The unit (u1) is a unit derived from the compound (m1) formed by polymerization of the compound (m1). The carbon-carbon unsaturated double bond in the Z group (the group of the same kind as the crosslinkable functional group) in the compound (m1) is lost by polymerization, and accordingly the unit (u1) has no crosslinkable functional group.

The proportion of the units (u1) in the polymer (C) is preferably from 10 to 90 weight%, more preferably from 15 to 90 weight%, further preferably from 20 to 90 weight%, particularly preferably from 50 to 90 weight%. When the proportion of the units (u1) is at least the lower limit value of the above range, the liquid repellency of the surface of the cured film will be more favorable. When it is at most the upper limit value of the above range, the polymer (C) is easily soluble in a solvent (E) for the coating composition.

### (Unit (u2))

The unit (u2) is a unit having a crosslinkable functional group and having no Cf group.

The crosslinkable functional group in the unit (u2) reacts with a crosslinkable functional group of the after-mentioned prepolymer (A) or compound (B), and they are integrated to form a cured film having high hardness and excellent solvent resistance.

The number of the crosslinkable functional group in the unit (u2) is preferably 1 in view of availability of the raw material and easiness of preparation.

The crosslinkable functional group in the unit (u2) is preferably a (meth)acryloyl(oxy) group in view of high reactivity with the crosslinkable functional group of the prepolymer (A) or the compound (B). The crosslinkable functional group in the compound (B) and the crosslinkable functional group in the polymer (C) which coexist in the curable composition may be the same or different from each other.

The polymerizable functional group (the group of the same kind as the crosslinkable functional group) which the monomer has is lost by polymerization, and accordingly the crosslinkable functional group of the unit (u2) is not the polymerizable functional group which the monomer has. Accordingly, the crosslinkable functional group of the unit (u2) is usually a crosslinkable functional group introduced e.g. by modification to a copolymer obtained by polymerization of the monomer.

The crosslinkable functional group of the unit (u2) is preferably introduced by a modification method of reacting a copolymer having reactive functional groups with a compound having a crosslinkable functional group. As the modification method, a know method may properly be used. Specifically, a compound having a polymerizable functional group and a reactive functional group (hereinafter sometimes referred to as "a compound (m4)") is copolymerized with the compound (m1) to obtain a copolymer having units having a reactive functional group (hereinafter sometimes referred to as "units (u4)"), which is reacted with a compound having a functional group reactive with the reactive functional group of the unit (u4) and a crosslinkable functional group (hereinafter sometimes referred to as "a compound (a2)") to obtain a polymer (C) having units (u2). The unit (u2) is a unit formed by reaction of the unit (u4) formed by polymerization of the compound (m4) and the compound (a2).

The reactive functional group may, for example, be a hydroxy group, an epoxy group or a carboxy group. In a case where the reactive functional group is a hydroxy group, the functional group reactive with the reactive functional group may, for example, be a carboxy group, an isocyanate group or acyl chloride. In a case where the reactive functional group is an epoxy group, the functional group reactive with the reactive functional group may, for example, be a carboxy group. In a case where the reactive functional group is a carboxy group, the functional group reactive with the reactive functional group may, for example, be a hydroxy group or an epoxy group.

As specific modification methods, for example, the following methods (i) to (vi) may be mentioned.
(i) A method of reacting a copolymer obtained by copolymerizing a monomer having a hydroxy group with an acid anhydride having a crosslinkable functional group.
(ii) A method of reacting a copolymer obtained by copolymerizing a monomer having a hydroxy group with a compound having an isocyanate group and a crosslinkable functional group.
(iii) A method of reacting a copolymer obtained by copolymerizing a monomer having a hydroxy group with a compound having an acyl chloride group and a crosslinkable functional group.
(iv) A method of reacting a copolymer obtained by copolymerizing an acid anhydride having a polymerizable functional group with a compound having a hydroxy group and a crosslinkable functional group.
(v) A method of reacting a copolymer obtained by copolymerizing a monomer having a carboxy group with a compound having an epoxy group and a crosslinkable functional group.
(vi) A method of reacting a copolymer obtained by copolymerizing a monomer having an epoxy group with a compound having a carboxy group and a crosslinkable functional group.

In a case where the copolymer having units (u4) is reacted with the compound (a2), the compound (a2) may be reacted to all the reactive functional groups of the copolymer, or may be reacted to some of the reactive functional groups of the copolymer. In the latter case, the obtained polymer (C) has units (u4) derived from the compound (m4). The polymer (C) to be used for the curable composition may have units (u4). Further, in a case where the reactive functional group of the unit (u4) may have unfavorable influences over the curable composition, with the reactive functional group of the unit (u4), a compound having a functional group reactive with the reactive functional group and having no crosslinkable functional group (hereinafter sometimes referred to as "a compound (b2)") may be reacted to convert the reactive functional group to an inert group.

The compound (b2) may, for example, ethyl isocyanate, propyl isocyanate, isopropyl isocyanate, butyl isocyanate, t-butyl isocyanate, hexyl isocyanate, cyclohexyl isocyanate, dodecyl isocyanate, octadecyl isocyanate, phenyl isocyanate, acetyl chloride, propionyl chloride, butyryl chloride, isobutyl chloride, pivaloyl chloride, isovaleryl chloride, valeryl chloride, 3,3-dimethylbutyryl chloride, hexanoyl chloride, heptanoyl chloride, 2-ethylhexanoyl chloride, octanoyl chloride, nonanoyl chloride, decanoyl chloride or lauroyl chloride, and is preferably ethyl isocyanate, propyl isocyanate, acetyl chloride or propionyl chloride.

A unit of which the reactive functional group is converted to an inert group will sometimes be referred to as "a unit (u5)".

The compound (m4) may, for example, be 2-hydroxyethyl (meth)acrylate or 4-hydroxybutyl (meth)acrylate in the method (i), (ii) or (iii). In the method (iv), it may, for example, be maleic anhydride, itaconic anhydride, citraconic anhydride or phthalic anhydride. In the method (v), it may, for example, be (meth)acrylic acid. In the method (vi), it may, for example, be glycidyl (meth)acrylate or 3,4-eopxycyclohexyl methyl acrylate.

The compound (a2) may, for example, be maleic anhydride, itaconic anhydride, citraconic anhydride or phthalic anhydride in the method (i). In the method (ii), it may, for example, be 2-(meth)acryloyloxyethyl isocyanate or 1,1-bis(acryloyloxymethyl)ethylisocyanate. In the method (iii), it may, for example, be (meth)acryloyl chloride or 3-butenoyl chloride. In the method (iv), it may, for example, be 2-hydroxyethyl (meth)acrylate or 4-hydroxybutyl (meth)acrylate. In the method (v), it may, for example, be glycidyl (meth)acrylate or 3,4-epoxycyclohexyl methyl acrylate. In the method (vi), it may, for example, be (meth)acrylic acid.

The unit (u2) is preferably a unit obtained by reacting a unit derived from a monomer having a hydroxy group with a compound having an isocyanate group and a crosslinkable functional group, or a unit obtained by reacting a unit derived from a monomer having a hydroxy group with a compound having an acyl chloride group and a crosslinkable functional group. Particularly preferred is a unit formed by reacting a unit derived from at least one monomer selected from the group consisting of 2-hydroxyethyl (meth)acrylate and 4-hydroxybutyl (meth)acrylate with at least one compound selected from the group consisting of (meth)acryloyl chloride, 2-methacryloyloxyethyl isocyanate and 2-acryloyloxyethyl isocyanate, whereby favorable reactivity with the prepolymer (A) will be obtained.

### (Unit (u3))

The polymer (C) may have units (u3) other than the units (u1) and the units (u2), as the case requires, within a range not to impair the effect to improve the liquid repellency. In a case where the polymer (C) has the units (u4) or the units (u5), these units are regarded as the units (u3).

The units (u3) are formed in the polymer (C) by polymerizing a compound having a polymerizable functional group and having no Cf group. This polymerizable compound may be the compound (m4) or may be a compound other than the compound (m4). Hereinafter such a polymerizable compound including the compound (m4) will be referred to as a compound (m3). The units (u3) are units formed in the polymer (C) by polymerizing the compound (m3), and as described above, the units (u4) and the (u5) are units included in the category of the units (u3).

The compound (m3) to give the units (u3) may, other than the compound (m4), for example, be a hydrocarbon olefin, a vinyl ether, an isopropenyl ether, an allyl ether, a vinyl ester, an allyl ester, a (meth)acrylate, a (meth)acrylamide, an aromatic vinyl compound, a chloroolefin, a conjugated diene or a fluorinated monomer other than the compound (m1). The compound (m3) may be used alone or in combination of two or more.

The compound (m3) may, for example, be specifically acrylic acid, methacrylic acid, methyl (meth)acrylate, ethyl (meth)acrylate, n-propyl (meth)acrylate, isopropyl (meth)acrylate, n-butyl (meth)acrylate, isobutyl (meth)acrylate, sec-butyl (meth)acrylate, tert-butyl (meth)acrylate, n-pentyl (meth)acrylate, 3-methylbutyl (meth)acrylate, n-hexyl (meth)acrylate, 2-ethyl-n-hexyl (meth)acrylate, n-octyl (meth)acrylate, n-dodecyl (meth)acrylate, cyclohexyl (meth)acrylate, isobornyl (meth)acrylate, (1,1-dimethyl-3-oxobutyl) (meth)acrylate, 2-acetoacetoxyethyl (meth)acrylate, 2-methoxyethyl (meth)acrylate, 2-ethoxyethyl (meth)acrylate, 2-(meth)acryloyloxyethyl succinate (for example, manufactured by SHIN-NAKAMURA CHEMICAL CO., LTD., tradename: SA), 3-(2H-benzotriazol-2-yl)-4-hydroxyphenethyl methacrylate (for example, manufactured by Otsuka Chemical Co., Ltd., tradename: RUVA-93), 2-(1H-benzo[d][1,2,3]triazole-1-carboxyamide)ethyl (meth)acrylate, styrene, (meth)acrylamide, N-vinylacetamide, N-vinylformamide, N-(1,1-dimethyl-3-oxobutyl) (meth)acrylamide, N-methoxymethyl (meth)acrylamide or N,N-bis(methoxymethyl) (meth)acrylamide. In view of availability, it is preferably acrylic acid, methacrylic acid, n-hexyl (meth)acrylate, cyclohexyl (meth)acrylate, isobornyl (meth)acrylate, n-octyl (meth)acrylate, n-dodecyl (meth)acrylate, 2-(meth)acryloyloxyethyl succinate, 3-(2H-benzotriazol-2-yl)-4-hydroxyphenethyl methacrylate or styrene.

The proportion of the units (u2) in the polymer (C) is preferably from 1 to 90 weight%, more preferably from 1 to 85 weight%, further preferably from 1 to 80 weight%, particularly preferably from 1 to 60 weight%, most preferably from 3 to 50 weight%. When the proportion of the units (u2) is at least the lower limit value of the above range, the reactivity with the prepolymer (A) or the compound (B) will be favorable. When it is at most the upper limit value of the above range, the liquid repellency of the surface of a cured film will be more favorable.

The proportion of the units (u3) in the polymer (C) is preferably at most 70 weight%, more preferably at most 60 weight%, particularly preferably at most 50 weight%. The lower limit value is preferably 0 weight%. When the proportion of the units (u3) is at most the upper limit value of the above range, sufficient proportions of the units (u1) and the units (u2) can be secured, and the liquid repellency of the surface of a cured film and the curing property of the curable composition will not be impaired.

In a case where the polymer (C) consists of the units (u1) and the units (u2), it is preferred that the content of the units (u1) is such an amount that the fluorine content in the polymer (C) is within the above preferred range, and the rest consists of the units (u2).

In a case where the polymer (C) consists of the units (u1), the units (u2) and the units (u3), it is preferred that the content of the units (u1) is such an amount that the fluorine content in the polymer (C) is within the above preferred range, the content of the units (u3) is within the above preferred range, and the rest consists of the units (u2).

### (Method for producing polymer (C))

The polymer (C) can be produced by polymerizing a monomer to obtain a copolymer, followed by the above modification as the case requires.

Polymerization of a monomer is preferably carried out in a solvent. Further, for polymerization of a monomer, a polymerization initiator is preferably used, and a chain transfer agent is preferably used as the case requires. When the monomer is stored, a polymerization inhibitor is preferably used as the case requires.

The solvent may, for example, be an alcohol (such as ethanol, 1-propanol, 2-propanol, 1-butanol or ethylene glycol), a ketone (such as acetone, 2-butanone, methyl isobutyl ketone or cyclohexanone), a cellosolve (such as 2-methoxyethanol, 2-ethoxyethanol or 2-butoxyethanol), a carbitol (such as 2-(2-methoxyethoxy)ethanol, 2-(2-ethoxyethoxy)ethanol, 2-(2-butoxyethoxy)ethanol), an ester (such as methyl acetate, ethyl acetate, n-butyl acetate, ethyl lactate, n-butyl lactate, ethylene glycol monomethyl ether acetate, propylene glycol monomethyl ether acetate, ethylene glycol diacetate or glycerin triacetate) or an ether (such as diethylene glycol dimethyl ether or diethylene glycol methyl ethyl ether). The solvent may be used alone or in combination of two or more.

The polymerization initiator may, for example, be a known organic peroxide, inorganic peroxide or azo compound. The organic peroxide and the inorganic peroxide may be used as a redox catalyst in combination with a reducing agent. The polymerization initiator may be used alone or in combination of two or more.

The organic peroxide may, for example, be benzoyl peroxide, lauroyl peroxide, isobutyryl peroxide, tert-butyl hydroperoxide or tert-butyl-α-cumyl peroxide.

The inorganic peroxide may, for example, be ammonium persulfate, sodium persulfate, potassium persulfate, hydrogen peroxide or a percarbonate.

The azo compound may, for example, be 2,2'-azobisisobutyronitrile, 1,1-azobis(cyclohexane-1-carbonitrile), 2,2'-azobis(2,4-dimethylvaleronitrile), 2,2'-azobis(4-methoxy-2,4-dimethylvaleronitrile), dimethyl 2,2'-azobisisobutyrate, or 2,2'-azobis(2-amidinopropane) dihydrochloride.

The chain transfer agent may, for example, be a known mercaptan or alkyl halide. The chain transfer agent may be used alone or in combination of two or more.

The mercaptan may, for example, be n-butyl mercaptan, n-dodecyl mercaptan, tert-butyl mercaptan, ethyl thioglycolate, 2-ethylhexyl thioglycolate or 2-mercaptoethanol.

The alkyl halide may, for example, be chloroform, carbon tetrachloride or carbon tetrabromide.

The polymerization inhibitor may be a known polymerization inhibitor.

The polymerization inhibitor may, for example, be specifically 2,6-di-tert-butyl-p-cresol.

In a case where the copolymer is modified also, the same solvent as above may be used. However, a solvent which may react with the compound (a2) is not used. Polymerization of the monomer is carried out in a solvent, and sequentially the compound (a2) is added and reacted to obtain the polymer (C).

Modification of the copolymer may be carried out in the presence of a catalyst or a neutralizing agent. For example, in a case where a copolymer having hydroxy groups is reacted with a compound having an isocyanate group and a crosslinkable functional group, a tin compound or the like may be used as a catalyst. The tin compound may, for example, be dibutyltin dilaurate, dibutyltin di(maleic acid monoester), dioctyltin dilaurate, dioctyltin di(maleic acid monoester) or dibutyltin diacetate. The tin compound may be used alone or in combination of two or more.

In a case where a copolymer having hydroxy groups is reacted with a compound having an acyl chloride group and a crosslinkable functional group, a basic catalyst may be used. The basic catalyst may, for example, be triethylamine, pyridine, dimethylaniline or tetramethylurea. The basic catalyst may be used alone or in combination of two or more.

The polymer (C) of the present invention contains units (u1) based on the compound (m1). Since the compound (m1) has a Cf group, the compound (m1) and the polymer (C) have favorable liquid repellency. Accordingly, the surface of a cured film obtained by curing a curable composition containing the polymer (C) repels water or oil, and even if water, oil or the like is attached thereto, it can easily be removed from the surface. The attached substance is not limited to a liquid, and may be a solid having an adherent surface. The polymer (C) imparts to the surface of a cured film obtained by curing a curable composition, properties to decrease the adhesion property and properties to make removal of the attached substance easy. The polymer (C) is useful as a non-adhesion imparting agent to be blended in a curable composition. For example, if an oily substance (particularly e.g. finger prints) such as sebum is attached to the surface of the cured film, the attached substance can easily be removed. Even in a case where such properties are utilized, as described above, a surface on which the properties are partially decreased can be formed.

Further, the compound (m1) is a compound in which R¹ in "a novel o-oxime photoinitiator" of the formula (I) disclosed in JP-A-2000-080068 is replaced with a R²-Cf group, and R⁵ is replaced with a [Z-(OCH₂CH₂)ₙ-(OC₆H₄)ₖ-]ₘX- group, and accordingly like "the novel o-oxime photoinitiator" disclosed in the document, decomposition occurs in its molecule by irradiation with ultraviolet light having a wavelength of from 350 to 370 nm even if no photocatalyst or the like is present, and a decomposition residue containing the Cf group can leave. That is, from the polymer (C), the Cf group present in the side chain is likely to leave by irradiation with ultraviolet light. Therefore, by partially irradiating the surface of a cured film obtained by curing a curable composition containing the polymer (C) with ultraviolet light, the liquid repellency of the portion irradiated with ultraviolet light of the surface of the cured film is decreased, and the portion may be converted to be relatively liquid-philic to the portion not irradiated with ultraviolet light. As described hereinafter, by irradiating the surface of a cured film obtained by curing a curable composition containing the polymer (C) with ultraviolet light via a photomask having a pattern, the portion irradiated with ultraviolet light can be converted to be liquid-philic, and a surface having a pattern of a liquid repellent region not irradiated with ultraviolet light and a liquid-philic region can be obtained.

### [Curable composition]

The curable composition of the present invention is a composition containing the polymer (C). As described hereinafter, it is a film made of a cured product obtained by heat-curing or photocuring the curable composition of the present invention. Accordingly, the curable composition of the present invention is a heat-curable composition or a photocurable composition.

The curable composition of the present invention preferably further contains a radical polymerization initiator (D) in view of the curing property. In view of the solvent resistance, the dielectric constant, etc. of an obtainable cured film, it is preferably a curable composition which further contains the prepolymer (A). In view of the hardness of an obtainable cured film, it preferably further contains the compound (B).

### (Prepolymer (A))

The prepolymer (A) has a polyarylene structure having a plurality of aromatic rings bonded via a single bond or a linking group, and has fluorine atoms and a crosslinkable functional group. By the curable composition containing the prepolymer (A), the dielectric constant of an obtainable cured film can be made low.

The crosslinkable functional group of the prepolymer (A) undergoes substantially no reaction at the time of producing the prepolymer (A), and undergoes radical polymerization reaction to cause crosslinking or chain extension between molecules of the prepolymer (A), by addition of an external energy in the presence of the radical polymerization initiation (D). Further, it is also reacted with the crosslinkable functional group of the compound (B) or the polymer (C) and integrated with it to produce a cured film. The crosslinkable functional group of the prepolymer (A) is preferably a vinyl group or an ethynyl group from the viewpoint that the reactivity at the time of producing the prepolymer (A) is low and that the reactivity in the presence of the radical polymerization initiator (D) is good.

The linking group in the polyarylene structure may, for example, be an ether bond (-O-), a sulfide bond (-S-), a carbonyl group (-CO-) or a sulfonyl group (-SO₂-). Among the prepolymers (A), a polymer having a structure in which aromatic rings are bonded by a liking group containing an ether bond (-O-) is referred to as "a fluorinated polyarylene ether prepolymer". The fluorinated polyarylene ether prepolymer is preferred in that it has an etheric oxygen atom, whereby the molecular structure has flexibility, and the flexibility of a cured film is good. The prepolymer (A) preferably comprises a fluorinated polyarylene ether prepolymer, particularly preferably consists solely of the fluorinated polyarylene ether prepolymer.

As a specific example of the linking group containing the ether bond, an ether bond (-O-) made solely of an etheric oxygen atom or an alkylene group containing an etheric oxygen atom in a carbon chain may, for example, be mentioned.

The prepolymer (A) has fluorine atoms. As it has fluorine atoms, the dielectric constant and the dielectric loss of a cured film tend to be low, such being desirable as a material to form an insulation film. When the dielectric constant and dielectric loss of an insulation film are low, it is possible to prevent delay of a signal propagation velocity and to obtain a device excellent in electrical properties. Further, as it has fluorine atoms, the water absorption of the cured film becomes low, whereby it is possible to prevent a change in the bonded state at the bonded electrodes and wiring portions therearound, or it is possible to prevent deterioration (such as rusting) of metals, and it presents a substantial effect to improve the reliability of a device.

As a specific example of the prepolymer (A) in the present invention, a prepolymer (hereinafter sometimes referred to as "a prepolymer (A1)") having a crosslinkable functional group and an ether bond, obtained by subjecting either one or both of a compound (x1) having a crosslinkable functional group and a phenolic hydroxy group and a compound (x2) having a crosslinkable functional group and a fluorinated aromatic ring, a compound (y) represented by the following formula (y), and a compound (z) having at least three phenolic hydroxy groups, to a condensation reaction in the presence of a hydrogen halide-removing agent, may be mentioned. 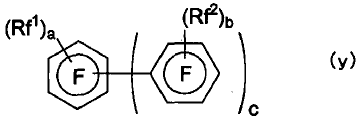 wherein c is an integer of from 0 to 3, a is an integer of from 0 to 3, b is an integer of from 0 to 3, Rf¹ is a fluoroalkyl group having at most 8 carbon atoms, provided that when there are a plurality of Rf¹'s, the plurality of Rf¹'s may be the same or different, Rf² is a fluoroalkyl group having at most 8 carbon atoms, provided that when there are a plurality of Rf²'s, the plurality of Rf²'s may be the same or different, and F in the aromatic ring represents that hydrogen atoms of the aromatic ring are all substituted by fluorine atoms.

As a preferred example of the compound (y), a compound wherein a is 0 or 1, b is 0 or 1, c is 0 or 1, Rf¹ is CF₃, and Rf² is CF₃, may be mentioned, and among them, perfluorobenzene, perfluorotoluene or perfluorobiphenyl is preferred.

The compound (z) may, for example, be 1,3,5-trihydroxybenzene, tris(4-hydroxyphenyl)methane, tris(4-hydroxyphenyl)ethane or 4-[4-[1,1-bis(4-hydroxyphenyl)ethyl]]-α,α-dimethylbenzylphenol, and is preferably 1,3,5-trihydroxybenzene or tris(4-hydroxyphenyl)ethane.

In the prepolymer (A1), by using the compound (z) having at least three phenolic hydroxy groups, it is possible to introduce branched structures to the polymer chain to make the molecular structure three dimensional thereby to increase the free volume of the polymer, whereby low densification i.e. a low dielectric constant can be accomplished. Further, usually, a linear chain polymer having aromatic rings is likely to undergo orientation of molecules due to stacking of the aromatic rings, but with the cured product of the present invention, orientation of molecules is suppressed by the introduction of branched structures, and consequently, the birefringence will be small.

The prepolymer (A1) may be produced by either or both of the following methods (i) and (ii).
(i) A method of subjecting the compound (y), the compound (z) and the compound (x1) to a condensation reaction in the presence of a hydrogen halide-removing agent.
(ii) A method of subjecting the compound (y), the compound (z) and the compound (x2) to a condensation reaction in the presence of a hydrogen halide-removing agent.

Further, in a case where the prepolymer (A1) is produced by both of the above (i) and (ii), the compound (y), the compound (z), the compound (x1) and the compound (x2) are subjected to a condensation reaction in the presence of a hydrogen halide-removing agent.

Moreover, in each of the above methods (i) and (ii), the condensation reaction may be a single stage reaction or a multi-step reaction. Further, among the reaction raw materials, a specific compound may be reacted preferentially in advance, and subsequently the other compounds may be reacted. In the case of the multi-step condensation reaction, an intermediate product obtained in the middle of the reaction may be separated from the reaction system, purified, and then used for a subsequent reaction (condensation reaction). In the reaction site, the raw material compounds may be charged all together, continuously or intermittently.

In the above process for producing the prepolymer (A1), the condensation reaction proceeds as represented by the following formula (1) in which an ether bond is formed by e.g. a reaction mechanism wherein a phenoxy group derived from a phenolic hydroxy group attacks the carbon atom to which a fluorine atom is bonded of the compound (y), and then the fluorine atom is eliminated. Further, in a case where the compound (z) and/or (x1) has two phenolic hydroxy groups which are in an ortho position to each other, there is a possibility that a dioxine skeleton is formed by the reaction represented by the following formula (2) by e.g. a similar reaction mechanism. From the viewpoint that the molecular structure has flexibility and the cured film has favorable flexibility, preferred is a prepolymer (A1) having no dioxine skeleton. That is, it is preferred that the compound (z) and/or the (x1) does not have two phenolic hydroxy groups which are in an ortho position to each other.

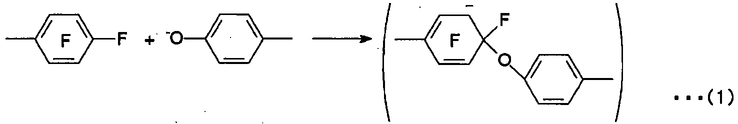

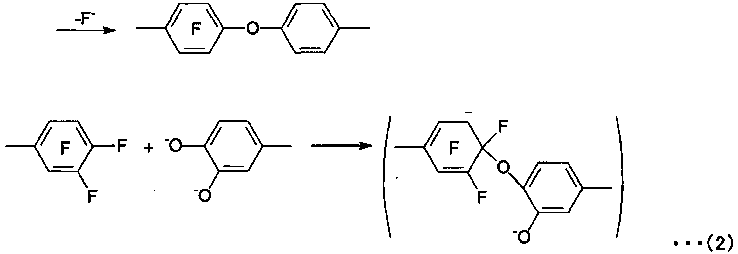

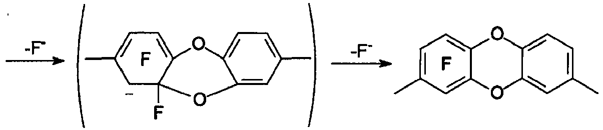

As the compound (x1) to be used in the production process (i), a compound (x11) having one phenolic hydroxy group and a compound (x12) having two phenolic hydroxy groups, are preferred.

Specific examples of the compound (x11) include a phenol having a reactive double bond such as 4-hydroxystyrene; and an ethynylphenol such as 3-ethynylphenol, 4-phenylethynyl phenol and 4-(4-fluorophenyl)ethynylphenol. They may be used alone or in combination as a mixture of two or more of them.

Specific examples of the compound (x12) include a bis(phenylethynyl)dihydroxybiphenyl such as 2,2'-bis(phenylethynyl)-5,5'-dihydroxybiphenyl and 2,2'-bis(phenylethynyl)-4,4'-dihydroxybiphenyl; and a dihydroxydiphenylacetylene such as 4,4'-dihydroxytolane and 3,3'-dihydroxytolane. They may be used alone or in combination as a mixture of two or more of them.

As the compound (x2) to be used in the production process (ii), a compound having a crosslinkable functional group and a perfluoroaromatic ring such as perfluorophenyl or perfluorobiphenyl, is preferred. Its specific examples include a fluorinated aryl having a reactive double bond, such as pentafluorostyrene, pentafluorobenzyl acrylate, pentafluorobenzyl methacrylate, pentafluorophenyl acrylate, pentafluorophenyl methacrylate, perfluorostyrene, pentafluorophenyl trifluorovinyl ether and 3-(pentafluorophenyl)pentafluoropropene-1; a fluorinated arylacetylene such as pentafluorophenylacetylene and nonafluorobiphenylacetylene; and a fluorinated diarylacetylene such as phenylethynylpentafluorobenzene, phenylethynylnonafluorobiphenyl and decafluorotolane. They may be used alone or in combination as a mixture of two or more of them. As the compound (x2), a fluorinated aryl having a double bond or a fluorinated arylacetylene having a triple bond is preferred since the crosslinking reaction thereby proceeds at a relatively low temperature, and the heat resistance of a prepolymer cured product thereby obtained becomes high.

As the hydrogen halide-removing agent to be used for the production of the prepolymer (A1), a basic compound is preferred, and an alkali metal carbonate, hydrogen carbonate or hydroxide is particularly preferred. Specific examples include sodium carbonate, potassium carbonate, sodium hydrogencarbonate, potassium hydrogencarbonate, sodium hydroxide and potassium hydroxide.

With respect to the amount of the hydrogen halide-removing agent to be used, in the production process (i), it is required to be an amount of at least equimolar, preferably from 1.1 to 3 times by the molar ratio to the total number of moles of phenolic hydroxy groups in the compound (z) and the compound (x1). In the production process (ii), it is required to be an amount of at least equimolar, preferably from 1.1 to 3 times by the molar ratio to the number of moles of phenolic hydroxy groups in the compound (z).

In the production processes (i) and (ii), the condensation reaction is preferably carried out in a polar solvent. The polar solvent is preferably an aprotic polar solvent such as an amide such as N,N-dimethylacetamide, N,N-dimethylformamide or N-methylpyrrolidone; a sulfoxide such as dimethylsulfoxide; a sulfone such as sulfolane; or an ether such as diethyl ether, tetrahydrofuran, dioxane, diethylene glycol dimethyl ether, diethylene glycol diethyl ether or triethylene glycol dimethyl ether.

To the polar solvent, toluene, xylene, benzene, tetrahydrofuran, benzotrifluoride, xylenehexafluoride or the like may be incorporated within a range not to deteriorate the solubility of the prepolymer (A1) to be formed and not to adversely affect the condensation reaction. By such incorporation, the polarity (the dielectric constant) of the solvent may be changed to control the reaction rate.

The condensation reaction conditions are preferably from 10 to 200°C for from 1 to 80 hours, more preferably from 20 to 180°C for from 2 to 60 hours, particularly preferably from 50 to 160°C for from 3 to 24 hours.

In the production process (i), the amount of the compound (z) to be used is preferably from 0.1 to 1 time, particularly preferably from 0.3 to 0.6 time in a molar ratio to the compound (y). The amount of the compound (x1) to be used is preferably from 0.1 to 2 times, particularly preferably from 0.2 to 1.5 times in a molar ratio to the compound (y).

In the production process (ii), the amount of the compound (z) to be used is preferably from 0.5 to 2 times, particularly preferably from 0.6 to 1.5 times in a molar ratio to the compound (y). The amount of the compound (x2) to be used is preferably from 0.1 to 2 times, particularly preferably from 0.2 to 1.5 times in a molar ratio to the compound (y).

When the respective amounts are in such ranges, the resulting prepolymer (A1) will have a low dielectric constant and high heat resistance, such being desirable.

As the production process of the prepolymer (A1), the production process (i) or (ii) may suitably be selected depending upon the physical properties such as the heat resistance, relative dielectric constant, birefringence, and flexibility, of a cured product obtainable after the curing. For example, in a case where the production process (ii) is used, the relative dielectric constant and birefringence values of a cured product obtainable by curing the prepolymer (A1) thus produced usually tend to be low. That is, to obtain a cured product having low relative dielectric constant and birefringence values, it is preferred to produce the prepolymer (A1) by the production process (ii).

After the condensation reaction or after formed into a solution, the prepolymer (A1) is purified by a method such as neutralization, reprecipitation, extraction or filtration. The purification is preferably carried out in a state where the polar solvent preferably used during the production, is present, or in a state as dissolved or dispersed in the after-mentioned solvent (E), since the efficiency is thereby good. In an application as an insulation film in electronic devices or an insulation film in multilayer wiring boards, a metal such as potassium or sodium derived from the hydrogen halide-removing agent and free halogen atoms are likely to cause operation failure of a transistor or corrosion of wiring, and accordingly, it is preferred to sufficiently carry out the purification.

Suitable examples of the prepolymer (A1) include a polymer obtainable by reacting a fluorinated aromatic compound (such as perfluoro(1,3,5-triphenylbenzene) or perfluorobiphenyl), a phenol compound (such as 1,3,5-trihydroxybenzene or 1,1,1-tris(4-hydroxyphenyl)ethane), anda crosslinkable functional group-containing aromatic compound (such as pentafluorostyrene, acetoxystyrene, chloromethylstyrene or pentafluorophenylacetylene), in the presence of a hydrogen halide-removing agent (such as potassium carbonate).

The number average molecular weight (Mn) of the prepolymer (A) is preferably from 1,000 to 100,000, particularly preferably from 5,000 to 50,000. When the number average molecular weight (Mn) is at least the lower limit value of the above range, the flexibility of the cured film does not tend to decrease. When it is at most the upper limit value of the above range, the curable composition can readily be purified.

The content of the prepolymer (A) in the curable composition of the present invention is preferably from 10 to 99.99 weight%, more preferably from 20 to 99.95 weight%, further preferably from 30 to 70 weight%, particularly preferably from 50 to 70 weight%. When it is at least the lower limit value of the above range, the dielectric constant of the cured film will be sufficiently low. Further, when it is at most the above upper limit value, the curable composition will easily be cured at low temperature, whereby the solvent resistance of the cured film will sufficiently be improved.

In a case where the curable composition contains the compound (B), the content of the prepolymer (A) is preferably from 20 to 90 parts by mass, more preferably from 30 to 85 parts by mass, particularly preferably from 40 to 80 parts by mass based on the total amount (100 parts by mass) of the prepolymer (A) and the compound (B).

### (Compound (B))

The compound (B) is a compound having a number average molecular weight (Mn) of from 140 to 5,000, having a crosslinkable functional group and having no fluorine atoms. By the curable composition containing the compound (B), a cured film having high hardness can be formed.

The number average molecular weight (Mn) of the compound (B) is preferably from 200 to 3,000, particularly preferably from 250 to 2,500. When the number average molecular weight (Mn) is at least the lower limit value of the above range, the compound (B) is less likely to be volatilized by heating. When it is at most the upper limit value of the above range, the viscosity of the compound (B) can be suppressed to be low, and therefore a uniform curable composition can readily be obtained when the compound (B) is mixed with the prepolymer (A).

The compound (B) has preferably at least 2 crosslinkable functional groups, whereby it is capable of intermolecular crosslinking, and more preferably has from 2 to 20, particularly preferably from 2 to 8 crosslinkable functional groups.

The crosslinkable functional groups of the compound (B) are preferably groups having no fluorine atoms and being reactive in the same step as in the step in which the crosslinkable functional group of the prepolymer (A) undergoes radical polymerization reaction.

The crosslinkable functional group of the compound (B) induces crosslinking or chain extension by the reaction of at least the crosslinkable functional groups with each other. Further, it is reacted with the crosslinkable functional group of the prepolymer (A) or the polymer (C), and they are integrated to form a cured film.

The crosslinkable functional group of the compound (B) is preferably a (meth)acryloyl(oxy) group from the viewpoint of high reactivity and availability, particularly preferably an acryloyl(oxy) group from the viewpoint of higher reactivity. Further, two or more different crosslinkable functional groups may be present in one molecule.

Further, each of the prepolymer (A), the compound (B) and the polymer (C) may have two or more different crosslinkable functional groups in one molecule. Further, the crosslinkable functional groups in the prepolymer (A), the compound (B) and the polymer (C) coexisting in the curable composition may be the same or different.

Specific examples of the compound (B) include dipentaerythritol triacrylate triundecylate, dipentaerythritol pentaacrylate monoundecylate, ethoxylated isocyanuric acid triacrylate, ε-caprolactone-modified tris-(2-acryloxyethyl) isocyanurate, 9,9-bis[4-(2-acryloyloxyethoxy)phenyl]fluorene, polyethylene glycol diacrylate, polyethylene glycol dimethacrylate, polypropylene glycol diacrylate, polypropylene glycol dimethacrylate, ethoxylated bisphenol A diacrylate, ethoxylated bisphenol A dimethacrylate, propoxylated bisphenol A diacrylate, propoxylated bisphenol A dimethacrylate, 1,10-decanediol diacrylate, 1,6-hexanediol diacrylate, 1,6-hexanediol dimethacrylate, 1,4-butanediol dimethacrylate, 1,3-butanediol dimethacrylate, hydroxypivalic acid neopentyl glycol diacrylate, 1,9-nonanediol diacrylate, 1,9-nonanediol dimethacrylate, neopentyl glycol diacrylate, neopentyl glycol dimethacrylate, pentaerythritol triacrylate, trimethylolpropane triacrylate, ethoxylated trimethylolpropane triacrylate, propoxylated trimethylolpropane triacrylate, triallyl cyanurate, triallyl isocyanurate, trimethallyl isocyanurate, 1,4-butanediol divinyl ether, 1,9-nonanediol divinyl ether, cyclohexane dimethanol divinyl ether, triethylene glycol divinyl ether, trimethylolpropane trivinyl ether, pentaerythritol tetravinyl ether, 2-(2-vinyloxyethoxy)ethyl acrylate, 2-(2-vinyloxyethoxy)ethyl methacrylate, trimethylolpropane diallyl ether, pentaerythritol triallyl ether, dipentaerythritol hexaacrylate, pentaerythritol tetraacrylate, an ethoxylated pentaerythritol tetraacrylate represented by the following formula (B-1), a propoxylated pentaerythritol tetraacrylate represented by the following formula (B-2), ditrimethylolpropane tetraacrylate, tricyclodecane dimethanol diacrylate, tricyclodecane dimethanol methacrylate, and a compound represented by the following formula (B-3).

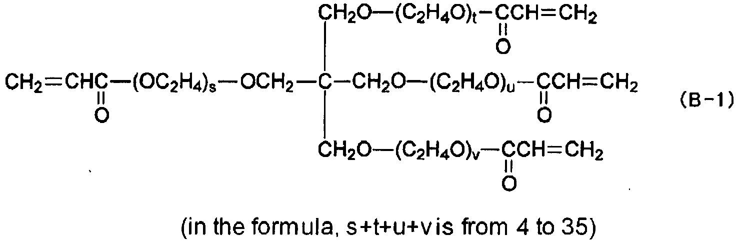

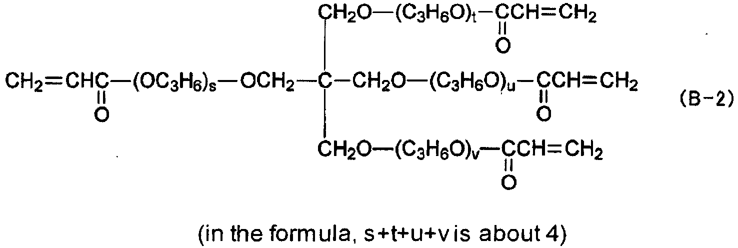

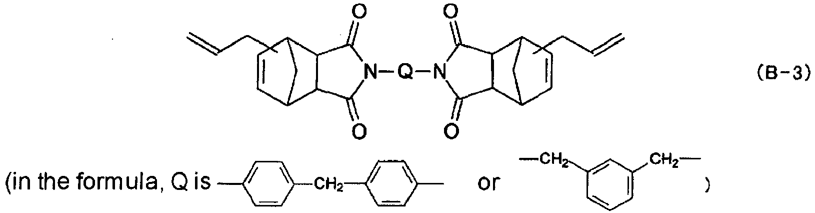

As the compound (B), polyester acrylates (a compound obtained by modifying both terminals of a condensate of a dihydric alcohol and a dibasic acid with acrylic acid, tradename: Aronix (M-6100, M-6200, M-6250 or M-6500), manufactured by TOAGOSEI CO., LTD.; and a compound obtained by modifying terminal hydroxy groups of a condensate of a polyhydric alcohol and a polybasic acid, with acrylic acid, tradename: Aronix (M-7100, M-7300K, M-8030, M-8060, M-8100, M-8530, M-8560 or M-9050) manufactured by TOAGOSEI CO., LTD.) may also be used. These products are commercially available.

As the compound (B) to be used in the present invention, ethoxylated isocyanuric acid triacrylate, 1,10-decanediol diacrylate, 1,9-nonanediol diacrylate, 1,9-nonanediol dimethacrylate, trimethylolpropane triacrylate, dipentaerythritol hexaacrylate, pentaerythritol tetraacrylate, ditrimethylolpropane tetraacrylate, tricyclodecane dimethanol diacrylate or ε-caprolactone modified tris-(2-acryloxyethyl)isocyanurate is preferred from the viewpoint of availability and reactivity.

In a case where the curable composition of the present invention contains the compound (B), its content is preferably from 10 to 80 parts by mass, more preferably from 15 to 70 parts by mass, particularly preferably from 20 to 60 parts by mass based on the total amount (100 parts by mass) of the prepolymer (A) and the compound (B). When the content is at least the lower limit value of the above range, the curable composition is likely to be cured at low temperature, whereby the solvent resistance of an obtainable cured film will sufficiently be improved. Such a composition can be applied to low temperature process using a substrate having low heat resistance. Further, in a case where the substrate has a large area, warpage of the substrate can be prevented. When it is at most the upper limit value of the above range, the dielectric constant of an obtainable cured film will sufficiently be low.

### (Polymer (C) in curable composition)

The polymer (C) in the curable composition is the above-described polymer of the present invention. By incorporating the polymer (C) to the curable composition, the liquid repellency of the surface of an obtainable cured film will be favorable.

The content of the polymer (C) in the curable composition of the present invention is preferably from 0.01 to 20 weight%, more preferably from 0.05 to 10 weight%, particularly preferably from 0.1 to 5 weight%. When the content is at least the lower limit value of the above range, the surface of the cured film will be excellent in the liquid repellency. When it is at most the upper limit value of the above range, the film physical properties of the cured film will be favorable.

In a case where the curable composition of the present invention contains the prepolymer (A) or the prepolymer (A) and the compound (B), the content of the polymer (C) is preferably from 0.1 to 20 parts by mass, particularly preferably from 0.2 to 15 parts by mass based on the total amount (100 parts by mass) of the prepolymer (A) and the compound (B).

### (Radical polymerization initiator (D))

The curable composition of the present invention may be heat-curable or photocurable. In a case where it is heat-curable, the curable composition contains a thermal polymerization initiator (D1) as the radical polymerization initiator (D), and in a case where it is photocurable, it contains a photopolymerization initiator (D2). The curable composition which is photocurable may be used as a negative photosensitive material.

As the thermal polymerization initiator (D1), a known initiator may be used. Specifically, it may, for example, be 2,2'-azobisisobutyronitrile, benzoyl peroxide, tert-butyl hydroperoxide, cumene hydroperoxide, di-tert-butyl peroxide or dicumyl peroxide. In view of the decomposition temperature, preferred is 2,2'-azobisisobutyronitrile or benzoyl peroxide. The thermal polymerization initiator (D1) may be used alone or in combination of two or more.

In a case where the photocurable composition of the present invention contains the thermal polymerization initiator (D1), its content is preferably from 0.1 to 20 weight%, particularly preferably from 1 to 15 weight%. When the content is at least the lower limit value of the above range, such a curable composition is likely to be cured at low temperature, whereby the solvent resistance of an obtainable cured film will sufficiently be improved. When it is at most the upper limit value of the above range, the storage stability of the curable composition will be favorable.

As the photopolymerization initiator (D2), a known initiator may be used. Specifically, it may, for example, be an oxime ester derivative (such as 1,2-octanedion, 1-[4-(phenylthio)-, 2-(o-benzoyloxime)] (for example, tradename: IRGACURE OXE01 manufactured by Ciba Specialty Chemicals Corporation), ethanone, 1-[9-ethyl-6-(2-methylbenzoyl)-9H-carbazol-3-yl]-, 1-(o-acetyloxime) (such as tradename: IRGACURE OXE02, manufactured by Ciba Specialty Chemicals Corporation), an α-aminoalkylphenone type compound (such as tradename: IRGACURE 369 or IRGACURE 907, manufactured by Ciba Specialty Chemicals Corporation), an acylphosphineoxide type compound (such as tradename: DAROCUR TPO, manufactured by Ciba Specialty Chemicals Corporation). From the viewpoint of reactivity of radicals to be generated, preferred is IRGACURE OXE01 or IRGACURE OXE02.

In a case where the curable composition of the present invention contains the photopolymerization initiator (D2), its content is preferably from 0.1 to 20 weight%, particularly preferably from 1 to 15 weight%. When the content is at least the lower limit value of the above range, such a composition is likely to be cured at low temperature, whereby the solvent resistance of an obtainable cured film will sufficiently be improved. When it is at most the upper limit value of the above range, the storage stability of the curable composition will be favorable.

### (Additives)

To the curable composition, an additive selected from various additives well known in the field of coating, for example, stabilizers (such as an ultraviolet absorber, an antioxidant, a thermal polymerization preventing agent, etc.), surfactants (such as a leveling agent, a defoaming agent, a precipitation-preventing agent, a dispersant, etc.), plasticizers and thickeners, may be incorporated, as the case requires, so as not to impair the effects of the present invention.

In a case where the cured film is a material (for example, an interlayer insulation film) which remains as a member which functions in a final product without being removed during the production process, an adhesion-improving agent (such as a silane coupling agent) may be added to the curable composition. It is preferred to incorporate an adhesion-improving agent to the curable composition, since the adhesion between the cured film of the curable composition and a layer adjacent thereto will be improved. It is possible to improve adhesion also by a method of preliminarily applying an adhesion-improving agent to the layer adjacent thereto.

In a case where the curable composition of the present invention contains an additive, its content is preferably from 0.0001 to 30 weight%, particularly preferably from 0.0001 to 20 weight%.

### (Preferred combination of curable composition)

Composition 1: A curable composition comprising the following prepolymer (A), compound (B), polymer (C) and thermal polymerization initiator (D1).

Prepolymer (A): A prepolymer obtained by condensing perfluorobiphenyl, 1,3,5-trihydroxybenzene and acetoxystyrene. The content of the prepolymer (A) in the curable composition is from 30 to 70 parts by mass.

Compound (B): At least one member selected from the group consisting of ethoxylated isocyanuric acid triacrylate, ε-caprolactone-modified tris-(2-acryloxyethyl) isocyanurate, 1,10-decanediol diacrylate, 1,9-nonanediol diacrylate, 1,9-nonanediol dimethacrylate, trimethylolpropane triacrylate, dipentaerythritol hexaacrylate, pentaerythritol tetraacrylate, ditrimethylolpropane tetraacrylate and tricyclodecanedimethanol diacrylate. The amount of the compound (B) is from 20 to 60 parts by mass based on the total amount (100 parts by mass) of the prepolymer (A) and the compound (B).

Polymer (C): A polymer having units (u1), units (u2) and units (u3), wherein the units (u1) are at least one member of the after-mentioned units (u1-1) to (u1-4), the units (u2) are the after-mentioned units (u2-1), and the units (u3) are at least one member of the after-mentioned units (u3-1) to (u3-9). The content of the polymer (C) in the curable composition is from 0.1 to 5 parts by mass.

Thermal polymerization initiator (D1): At least one member selected from the group consisting of benzoyl peroxide and 2,2'-azobisisobutyronitrile. The content of the thermal polymerization initiator (D1) in the curable composition is from 1 to 15 parts by mass.

Combination 2: A curable composition comprising the following prepolymer (A), compound (B), polymer (C) and photopolymerization initiator (D2).

Prepolymer (A): A prepolymer obtained by condensing perfluorobiphenyl, 1,3,5-trihydroxybenzene and acetoxystyrene. The content of the prepolymer (A) in the curable composition is from 30 to 70 parts by mass.

Compound (B): At least one member selected from the group consisting of ethoxylated isocyanuric acid triacrylate, ε-caprolactone-modified tris-(2-acryloxyethyl) isocyanurate, 1,10-decanediol diacrylate, 1,9-nonanediol diacrylate, 1,9-nonanediol dimethacrylate, trimethylolpropane triacrylate, dipentaerythritol hexaacrylate, pentaerythritol tetraacrylate, ditrimethylolpropane tetraacrylate and tricyclodecanedimethanol diacrylate. The amount of the compound (B) is from 20 to 60 parts by mass based on the total amount (100 parts by mass) of the prepolymer (A) and the compound (B).

Polymer (C): A copolymer having units (u1), units (u2) and units (u3), wherein the units (u1) are at least one member of the after-mentioned units (u1-1) to (u1-4), the units (u2) are the after-mentioned units (u2-1), and the units (u3) are at least one member of the after-mentioned units (u3-1) to (u3-9). The content of the polymer (C) in the curable composition is from 0.1 to 5 parts by mass.

Photopolymerization initiator (D2): At least one member selected from the group consisting of IRGACURE OXE01 (manufactured by Ciba Specialty Chemicals Corporation), IRGACURE OXE02 (manufactured by Ciba Specialty Chemicals Corporation), IRGACURE 369 (manufactured by Ciba Specialty Chemicals Corporation), IRGACURE 907 (manufactured by Ciba Specialty Chemicals Corporation) and DAROCUR TPO (manufactured by Ciba Specialty Chemicals Corporation). The content of the photopolymerization initiator (D2) in the curable composition is from 1 to 15 parts by mass.

### [Coating composition]

The coating composition of the present invention contains the above curable composition and a solvent (E). The coating composition is used to form a film of the curable composition by applying the coating composition on the surface of a substrate to form a coating film, and removing the solvent (E) from the coating film. Removal of the solvent (E) is usually carried out by evaporating the solvent (E). Accordingly, it is necessary that the solvent (E) has a boiling point lower than those of components in the curable composition. Among the above components (A) to (D), a compound having the lowest boiling point is usually the compound (B), and therefore in a case where the curable composition contains the compound (B), a solvent (E) having a boiling point lower than that of the compound (B) is used. In other words, as the compound (B), it is preferred to use a compound having a boiling point sufficiently higher than that of the solvent (E) to be used.

Further, a film of the curable composition containing no solvent (E), formed by removing the solvent (E) from the coating film of the coating composition will sometimes be referred to as "a dry film". The dry film is a film of the curable composition which is not cured. Further, a film of the curable composition which is cured, formed by curing the curable composition of the dry film will sometimes be referred to as "a cured film".

As the solvent (E), a known solvent may be used. A ketone type solvent, an ester type solvent, an ether type solvent, an amide type solvent or an aromatic type solvent may, for example, be mentioned, and specific examples include polypropylene glycol monomethyl ether acetate (hereinafter sometimes referred to as "PGMEA"), mesitylene, N,N-dimethylacetamide, cyclohexanone and tetrahydrofuran.

The content of the solvent (E) in the coating composition is preferably from 1 to 99.995 weight%, more preferably from 30 to 99.99 weight%, particularly preferably from 50 to 90 weight%.

### (Preferred combination of coating composition)

As the coating composition of the present invention, the following combinations are preferred.

Combination 3: A coating composition comprising the above preferred combination 1 of the curable composition and the following solvent (E).

Solvents (E): At least one member selected from the group consisting of PGMEA and cyclohexanone. The content in the coating composition is from 50 to 90 weight%.

Combination 4: A coating composition comprising the above preferred combination 2 of the curable composition and the above solvent (E) in the preferred combination 3 of the coating composition.

Further, in a case where the curable composition of the present invention is a curable composition containing the prepolymer (A), the compound (B), the polymer (C) and the radical polymerization initiator (D), it can be sufficiently cured by heating to at most 250°C (preferably at most 200°C). Accordingly, such a coating composition can be applied to a process (low temperature process) in which the upper limit value of the heating temperature is at most 250°C. Further, from such a coating composition, a cured film having excellent solvent resistance, a low dielectric constant and favorable liquid repellency on its surface can be formed.

### [Article having cured film]

The article having a cured film of the present invention is an article comprising a substrate, and a cured film obtained by curing the curable composition of the present invention formed on the surface of the substrate.

"The cured film obtained by curing the curable composition of the present invention formed on the surface of the substrate" includes both a case where the cured film is directly formed on the surface of a substrate and a case where an optional layer is formed on the surface of a substrate and the cured film is formed on the optional layer.

### (Substrate)

A material for the substrate may, for example, be plastic, glass or silicon. It is preferred to use plastic such as polycarbonate, polyethylene terephthalate, polyethylene naphthalate, polyethersulfone or polyimide, in view of excellent mechanical flexibility.

### (Cured film)

The thickness of the cured film may properly be set depending upon the purpose of use, and is usually at a level of from 0.1 to 100 µm, preferably from 0.2 to 50 µm.

### [Process for producing article having cured film]

The process for producing an article having a cured film of the present invention is a process comprising the following steps (I) and (II).
(I) A step of applying the coating composition of the present invention on the surface of a substrate to form a coating film, and removing the solvent (E) to form a dry film.
(II) A step of heating the dry film obtained in the above step (I) or irradiating it with light to form a cured film.

"Applying the coating composition of the present invention on the surface of a substrate to form a coating film, and removing the solvent (E) to form a cured film" includes both a case where the dry film is directly formed on the surface of a substrate and a case where an optional layer is formed on the surface of a substrate and the dry film is formed on the surface of the optional layer.

In a case where the curable composition is used without using the coating composition, the step (I) is as follows.
(I) A step of applying the curable composition of the present invention on the surface of a substrate to form a coating film, thereby to form a dry film.

### (Step (I))

The application method is not particularly limited so long as a uniform coating film can be formed. For example, a spin coating method, a wipe coating method, a spray coating method, a squeegee coating method, a dip coating method, a die coating method, an ink jet method, a flow coating method, a roll coating method, a casting method, a slit coating method, a screen printing method, a Langmuir-Blodgett method or a gravure coating method may be mentioned. In view of the productivity, a spin coating method, an ink jet method or a slit coating method is preferred.

As a method of removing the solvent (E) in the coating film, a known method is mentioned, and a method by heating, a method by reducing pressure or a method by heating and reducing pressure may, for example, be mentioned. From the viewpoint that a defect is less likely to occur in the coating film, a method by heating is preferred. A heating temperature is preferably from 30 to 200°C, particularly preferably from 40 to 150°C.

### (Step (II))

In a case where the curing is carried out by heating (heat-curing), the coating composition is applied on the surface of a substrate to form a coating film, a heating step (prebaking) for the purpose of removing the solvent (E) is carried out, and further a heating step (curing step) is carried out to obtain a cured film. In the case of heat-curing, the heating step for curing may also function as the heating step for removing the solvent. A heating temperature of the heating step (prebaking) is preferably from 40 to 200°C, particularly preferably from 60 to 200°C. A heating temperature of the heating step (curing step) is preferably from 100 to 200°C, particularly preferably from 120 to 200°C. A heating time of the heating step (prebaking) is preferably from 1 to 10 minutes, particularly preferably from 1 to 5 minutes. A heating time of the heating step (curing step) is preferably from 1 to 10 minutes, particularly preferably from 1 to 5 minutes. Here, "the heating temperature is at most 200°C" means that the temperature of an article to be heated does not exceeds 200°C. Substantially, a temperature of a heating device such as a hotplate or an oven may be set to at most 200°C.

In a case where the curing is carried out by irradiation with light (photocuring), the coating composition is applied on the surface of a substrate to form a coating film, a heating step (prebaking) for the purpose of removing the solvent (E) is carried out, then the film is irradiated (exposed) with light, and as the case requires, a heating step (curing step) is carried out to obtain a cured film.

Light to be applied for curing the film is not particularly limited so long as it is light having a wavelength to which the photopolymerization initiator (D2) contained in the curable composition has a sensitivity. Usually, light to be used for curing is ultraviolet light (wavelength: 200 to 400 nm, preferably 300 to 400 nm), but is not limited thereto. Further, it is preferred not to use light having a wavelength with which decomposition occurs in the side chains of the units (u1) in the polymer (C), and the photopolymerization initiator (D2) having a sensitivity to light having such a wavelength.

A heating temperature of the heating step (prebaking) in the case of photocuring is preferably from 30 to 100°C, particularly preferably from 40 to 100°C. A heating temperature of the heating step (curing step) is preferably from 60 to 200°C, particularly preferably from 100 to 200°C. A heating time of the heating step (prebaking) is preferably from 1 to 20 minutes, particularly preferably from 1 to 10 minutes.

A heating time of the heating step (curing step) is preferably from 1 to 20 minutes, particularly preferably from 1 to 10 minutes.

In the case of carrying out micro-fabrication by photolithography, by selective irradiation (exposure) with light using a photomask or laser, the irradiated portion (exposed portion) is cured. Accordingly, after the exposure, development (a step of dissolving or dispersing the non-exposed portion in a solvent and removing it) is carried out to remove the non-exposed portion, and the remaining solvent in the cured portion is removed to obtain a micro-fabricated cured film. As the case requires, after the development, a heating step (curing step) may be carried out. In such a case, the remaining solvent can be removed in this heating step (curing step). Further, after the exposure and before the development, as the case requires, a heating step (post-exposure baking) may be carried out. The heating temperature of the heating step (post-exposure baking) is preferably from 60 to 200°C, particularly preferably from 100 to 200°C. The heating time of the heating step (post-exposure baking) is preferably from 1 to 20 minutes, particularly preferably from 1 to 10 minutes.

### [Process for producing article having pattern of liquid-philic region and liquid repellent region]

The article having a pattern of a liquid-philic region and a liquid repellent region of the present invention is produced by either of a process of irradiating the cured film with ultraviolet light to convert the irradiated portion to be a liquid-philic surface, and a process of irradiating the dry film with ultraviolet light to convert the irradiated portion to be liquid-philic, and then curing the dry film to form a cured film.

That is, the former process is a process of forming a dry film on the surface of a substrate, curing the dry film to form a cured film, and partially irradiating the surface of the cured film with ultraviolet light to form a pattern of a liquid-philic region and a liquid repellent region on the surface of the cured film. The latter process is a process of forming a dry film on the surface of a substrate, partially irradiating the surface of the dry film with ultraviolet light to form a pattern of a liquid-philic region and a liquid repellent region on the surface of the dry film, and curding the dry film.

Specifically, the article having a pattern of a liquid-philic region and a liquid repellent region may be produced by carrying out the following step (IIα) or (IIβ) as the step (II) after the above step (I). That is, by carrying out the step (IIα) or (IIβ), patterning of a liquid-philic region and a liquid repellent region can be carried out.

(IIα) A step of heating the dry film obtained in the step (I) or irradiating the dry film with light to form a cured film, and partially irradiating the surface of the cured film with ultraviolet light to obtain an article having a pattern of a liquid-philic region and a liquid repellent region on the surface of the cured film.

(IIβ) A step of partially irradiating the surface of the dry film obtained in the step (I) with ultraviolet light to form a pattern of a liquid-philic region and a liquid repellent region on the surface of the dry film, and heating the dry film having the pattern formed thereon or irradiating it with light to obtain an article having a pattern of a liquid-philic region and a liquid repellent region on the surface of the cured film.

The conditions (temperature, time) under which the dry film is heated or irradiated with light to form a cured film in the step (IIα) and the conditions (light, temperature, time) under which the dry film having a pattern formed thereon is cured by heating or light irradiation in the step (IIβ), are as described in the step (II).

Hereinafter, a portion not irradiated with ultraviolet light will be referred to as a liquid repellent region, and a portion converted to be liquid-philic by irradiation with ultraviolet light will be referred to as a liquid-philic region.

To form a pattern of a liquid-philic region and a liquid repellent region, a method of irradiating the surface of the dry film or the cured film with ultraviolet light via a photomask, or a method of selectively irradiating the surface of the dry film or the cured film with ultraviolet light by using laser, may be mentioned.

As a light source of the ultraviolet light, a light source which can apply ultraviolet light having a wavelength of at least 300 nm such as a high-pressure mercury lamp (i-line: 365 nm) or a YAG laser (third harmonic: 355 nm) may be used. Since the surface of the dry film or the cured film may be converted to be liquid-philic also by ultraviolet light having a wavelength less than 300 nm, a light source which can apply ultraviolet light having a wavelength less than 300 nm may be used.

After the step (IIα) or (IIβ), a decomposition residue containing the Cf group may be removed. For example, it may be removed by heating or in vacuum.

### [Article having functional thin film]

In the article having a pattern of a liquid-philic region and a liquid repellent region of the present invention, a functional thin film may be formed on the surface of the liquid-philic region.

As an embodiment of the functional thin film, an electrode (thin-film transistor, organic EL device), a semiconductor layer (thin-film transistor, organic EL device), a conductor layer (printed circuit board, multilayer wiring, touch panel, solar cell), a transistor material or a resin layer may, for example, be mentioned. Since the cured film is insulating, preferred is a thin-film transistor in which the functional thin-film is an electrode.

As the semiconductor layer, an organic semiconductor, an oxide semiconductor or a silicon semiconductor may be mentioned.

As the resin layer, a thermosetting resin such as a phenol resin, a urea resin, a melamine resin, an acrylic resin, an epoxy resin, a polyurethane, a polyester, a silicone resin or a polyimide, a photocurable resin or the prepolymer (A) may, for example, be mentioned.

### (Organic thin-film transistor)

Now, an example of an organic thin-film transistor will be described with reference to the drawing. However, the functional thin film of the present invention is by no means restricted to the following organic thin-film transistor.

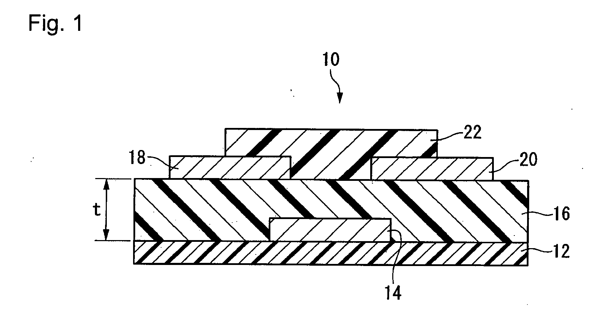

Fig. 1 is a cross-sectional view illustrating an example of an organic thin-film transistor.

An organic thin-film transistor 10 comprises a substrate 12; a gate electrode 14 formed on the surface of the substrate 12; a gate insulation film 16 (cured film) covering the surface of the gate electrode 14 and the substrate 12; a source electrode 18 and a drain electrode 20 (functional thin film) selectively formed on the surface of the gate insulation film 16; and an organic semiconductor layer 22 formed on the surface of the source electrode 18 and the drain electrode 20, and the gate insulation film 16 between the electrodes.

As a material of the substrate 12, the above-described material may be mentioned, and preferred embodiments of the substrate 12 are also the same.

The gate electrode 14, the source electrode 18 and the drain electrode 20 are formed by a conductor. The conductor may, for example, be silicon, doped silicon, platinum, gold, silver, copper, chromium, aluminum, calcium, barium, indium tin oxide, indium zinc oxide, zinc oxide, carbon black, a fullerene, carbon nanotubes, polythiophene, polyethylene dioxythiophene, a polystyrenesulfone, polyaniline, polypyrrole or polyfluorene. The conductors may be used alone or in combination of two or more. The materials of the gate electrode 14, the source electrode 18 and the drain electrode 20 may be the same or different.

The gate insulation film 16 is made of a cured film obtained by forming a dry film on the surface of the gate electrode 14 and the substrate 12, and curing the dry film.

The thickness (the thickness t at a portion where the gate electrode 14 is not present) of the gas insulation film 16 is preferably from 1 nm to 10 µm, more preferably from 2 nm to 5 µm, particularly preferably from 5 nm to 1 µm. When the thickness of the gate insulation film 16 is at least the lower limit value of the above range, leakage current is less likely to occur between the gate electrode 14 and the source electrode 18. When it is at most the upper limit value of the above range, the driving voltage can be suppressed.

As the material of the organic semiconductor layer 22, a known low molecular weight compound, oligomer or polymer may, for example, be mentioned.

The low molecular weight compound may, for example, be pentacene, rubrene, phthalocyanine, perylene, fullerene or a derivative thereof.

The oligomer may, for example, be oligothiophene or a derivative thereof.

The polymer may, for example, be poly-p-phenylenevinylene (PPV), polyfluorene, a fluorene/benzothiadiazole copolymer, a fluorene/triphenylamine copolymer, a fluorene/dithiophene copolymer, polythiophene, polyaniline, polyacetylene, polypyrrole or derivative thereof.

The thickness of the organic semiconductor layer 22 is preferably from 5 nm to 100 µm, more preferably from 10 nm to 10 µm, particularly preferably from 10 nm to 1 µm.

In the organic thin-film transistor 10, by forming the gate insulation film 16 using the curable composition of the present invention, the leakage current is reduced. Further, since it is possible to make the gate insulation film 16 thin, downsizing of a device can be realized, and the driving voltage of the transistor can be decreased.

Further, in the organic thin-film transistor 10, since the surface of the gate insulation film 16 has favorable liquid repellency, such effects will be obtained that molecules in the organic semiconductor layer 22 provided on the gate insulation film 16 are likely to be aligned, polar groups to be top sites of a carrier are less likely to be present on the surface, and moisture and the like in the air are less likely to be adsorbed. Accordingly, the electron mobility in the organic thin-film transistor 10 will be high, and the stability and the reliability will improve.

### [Process for producing article having functional thin film]

An article having a functional thin film is produced by a process comprising the above process (I) and process (IIα) or (IIβ) and the following step (III).
(III) A step of selectively depositing a composition for forming a functional thin film to the surface of the above liquid-philic region (the portion of the cured film irradiated with ultraviolet light) to form a functional thin film, after the above step (IIα) or (IIβ).

The composition for forming a functional thin film is a composition for forming e.g. an electrode, a semiconductor layer, a conductor layer, a transistor material or a resin layer, and will be hereinafter sometimes referred to as "a composition for an electrode, a composition for a semiconductor layer, a composition for a conductor layer, a composition for a transistor material or a composition for a resin layer".

The composition for forming a functional thin film may, for example, in a case where the functional film is an electrode, be a coating fluid containing the above conductor or a precursor of the conductor.

"Selectively depositing a composition for forming a functional thin film to the surface of the liquid-philic region (the portion of the cured film irradiated with ultraviolet light) to form a functional thin film" includes both a case where a functional thin film is directly formed selectively on the surface of the cured film and a case where an optional layer is formed selectively on the surface of the cured film and the cured film is formed on the surface of the optional layer.

If the composition for forming a functional thin film is applied to the surface of the cured film having a pattern of a liquid-philic region and a liquid repellent region, the composition for forming a functional thin film is selectively deposited to the liquid-philic region and will not be deposited to the liquid repellent region. Accordingly, a functional thin film (such as an electrode) having a predetermined pattern can easily be formed only on the surface of the liquid-philic region of the cured film. To form the composition for forming a functional thin film deposited to the surface of the liquid-philic region of the cured film into a functional thin film, a known method may be employed.

A functional thin film may further be formed on the surface of the liquid repellent region of the cured film. In such a case, in order to improve the film-forming property of the functional thin film, the entire surface may be exposed without using a photomask to convert the liquid repellent region to be liquid-philic, and then the composition for forming a functional thin film to form a functional thin film may be applied to form a functional thin film.

### (Process for producing organic thin-film transistor)

Now, an example of the process for producing an organic thin-film transistor will be described with reference to the drawing. Further, the process for producing an article having a functional thin film of the present invention is a process comprising the above steps (I), (IIα) or (IIβ) and (III) and is not limited to the following process for producing an organic thin-film transistor.

### Step (I):

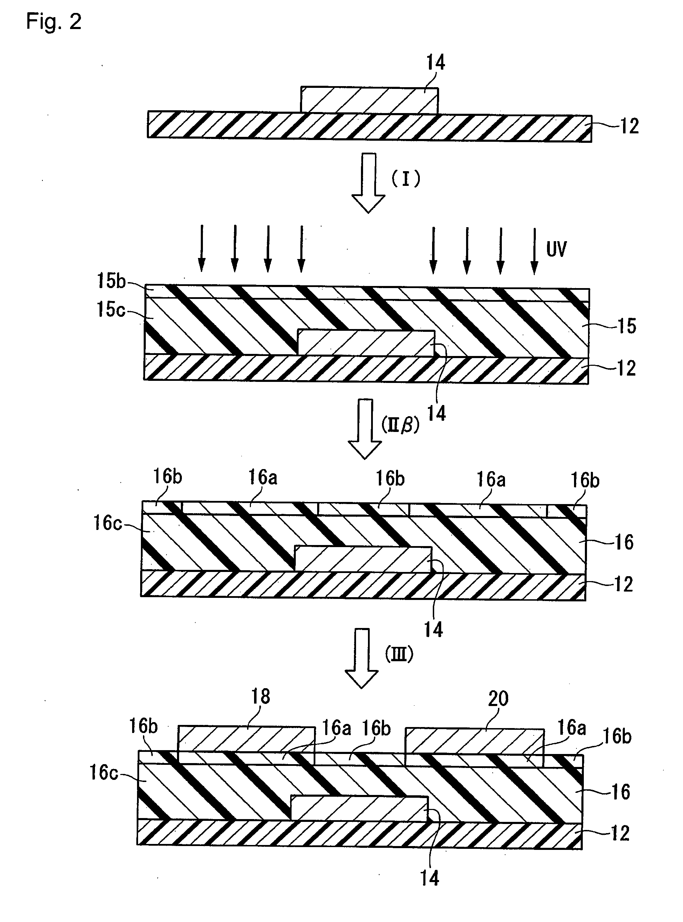

As shown in Fig. 2, a substrate 12 having a gate electrode 14 formed on its surface is prepared.

As a method for forming the gate electrode 14, sputtering, vacuum deposition, spin coating, spray coating, printing or ink jet may, for example, be mentioned.

Then, the coating composition of the present invention is applied to the surface of the gate electrode 14 and the substrate 12, and the solvent (E) is removed to form a dry film 15. It is considered that in the dry film 15, structures derived from the prepolymer (A) gather on the substrate 12 side, and the Cf groups are unevenly present on the side opposite from the substrate 12. That is, the surface of the dry film 15 is a liquid repellent region 15b having liquid repellency. The reference symbol 15c in the drawing illustrates an internal region other than the surface of the gate insulation film 16.

### Step (IIβ):

As shown in Fig. 2, the surface of the dry film 15 obtained in the step (I) is partially irradiated with ultraviolet light (UV) using a photomask (not shown) or laser (not shown) to make at least some of the Cf groups on the surface of the dry film 15 leave to form a liquid-philic region (not shown). In such a manner, a pattern of the liquid-philic region (not shown) and the liquid repellent region 15b is formed. It is considered that in the dry film 15, the liquid-philic region (not shown) and the liquid repellent region 15b are not distinctly separated from the internal region 15c below their surfaces having surface properties, but the concentration of the Cf groups varies continuously in the thickness direction.

As shown in Fig. 2, the dry film 15 having a pattern formed thereon is heated or irradiated with light to form a gate insulation film 16 (cured film) having a pattern comprising a liquid-philic region 16a and a liquid repellent region 16b. It is considered that in the gate insulation film 16, the liquid-philic region 16a and the liquid repellent region 16b are not distinctly separated from the internal region 16c below their surfaces having surface properties, but the concentration of the Cf groups varies continuously in the thickness direction.

### Step (III):

A coating fluid containing a conductor or a precursor of the conductor is applied to the surface of the gate insulation film 16 (cured film) having a pattern of the liquid-philic region 16a and the liquid repellent region 16b, whereupon the coating fluid is selectively deposited to the liquid-philic region 16a and is not deposited to the liquid repellent region 16b. Accordingly, as shown in Fig. 2, a source electrode 18 and a drain electrode 20 having a predetermined pattern can easily be formed only on the liquid-philic region 16a of the gate insulation film 16.

As a method of applying the coating fluid, ink jet, a dispenser, printing or the like may be mentioned.

### Step (IV):

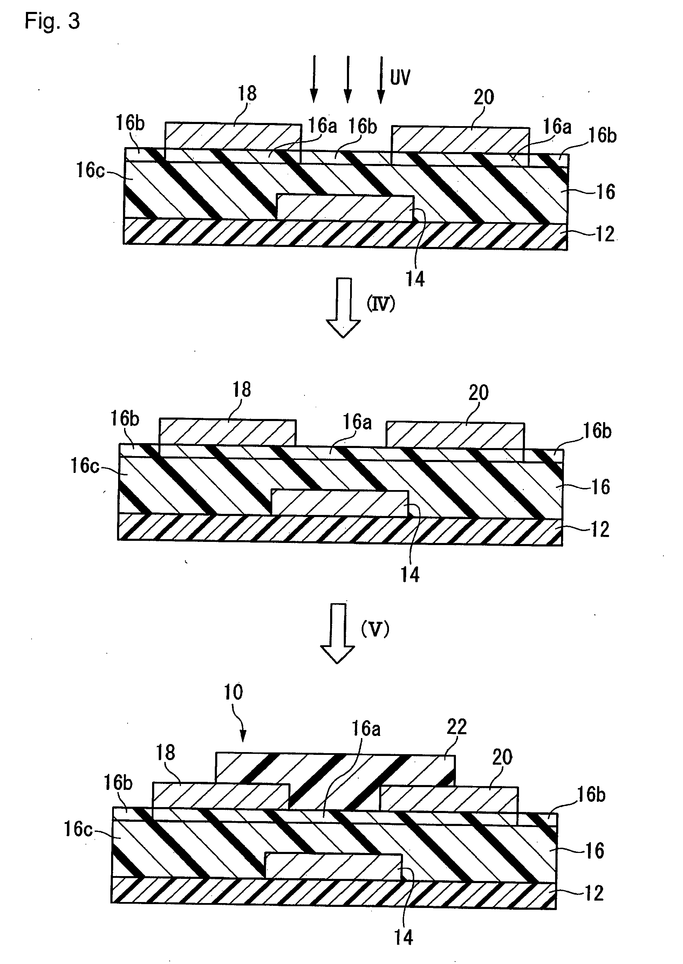

As shown in Fig. 3, at least the liquid repellent region 16b of the gate insulation film 16 between the source electrode 18 and the drain electrode 20 is irradiated with ultraviolet light (UV) to make at least some of the Cf groups on the surface of the gate insulation film 16 leave to convert the surface of the gate insulation film 16 between the electrodes to a liquid-philic region 16a. By converting the surface of the gate insulation film 16 between the electrodes to a liquid-philic region 16a, the following composition for forming an organic semiconductor layer can be applied to the surface of the gate insulation film 16 between the electrodes.

### Step (V):

As shown in Fig. 3, a composition for forming an organic semiconductor layer is applied to the surface of the source electrode 18 and the drain electrode 20, and the gate insulation film 16 between the electrodes, and an organic semiconductor layer 22 is formed by a known method such as heating. Further, a method of forming a layer made of a precursor of an organic semiconductor and then converting the precursor to an organic semiconductor by applying light or heat may also be employed. The precursor may, for example, be silylethyne-substituted pentacene or a tetrabicycloporphyrin derivative. The precursor may be converted to pentacene or a tetrabenzoporphyrin derivative by heating.

### EXAMPLES

Now, the present invention will be described in further detail with reference to Examples. However, it should be understood that the present invention is by no means restricted to such specific Examples.

Example 1 is a Preparation Example and Examples 2-1 to 2-4, 3-1 to 3-20, 4-1 to 4-20 and 5-1 to 5-20 are Examples of the present invention.

### (PGMEA contact angle)

The contact angle of the surface of the cured film was measured by a droplet method at 25°C using a contact angle meter CA-A, tradename, manufactured by Kyowa Interface Science Co., Ltd. About 1 µL of PGMEA was dropped on the cured film, and the contact angle was measured.

### [Example 1]

### (Preparation of prepolymer (A1-1))

Into a 10 L (liter) glass four-necked flask equipped with a Dimroth condenser, a thermocouple thermometer and a mechanical stirrer, perfluorobiphenyl (650 g), 1,3,5-trihydroxybenzene (117 g) and N,N-dimethylacetamide (hereinafter sometimes referred to as "DMAc") (6,202 g) were charged. The flask was heated on an oil bath with stirring, and at the time when the liquid temperature reached 60°C, sodium carbonate (575 g) was quickly added. While stirring was continued, the mixture was heated at 60°C for 24 hours. Then, the mixture was cooled to 0°C with stirring, 4-acetoxystyrene (200 g) and potassium hydroxide (532 g) were added, followed by stirring at 0°C for 24 hours, and the mixture was gradually dropwise added to about 10 L of a 5N aqueous hydrochloric acid solution for reprecipitation. The precipitate was collected by filtration and washed twice with pure water. Then, the precipitate was vacuum-dried at 60°C for 12 hours to obtain a white powdery prepolymer (A1-1) (800 g). The number average molecular weight (Mn) of the prepolymer (A-1) was 10,000.

### [Example 2-1]

### (Preparation of compound (m1-1))

A reaction represented by the following formula was carried out. 17.7 g of 4-hydroxybenzenethiol and 20.0 g of 4'-bromopropiophenone were dissolved in 151 g of DMAc to obtain a solution. While the solution was stirred at 80°C, 25.9 g of potassium carbonate was added to the solution, followed by stirring at 80°C for 12 hours to obtain a solution of compound (a1-1). Then, the obtained solution of compound (a1-1) was poured into water, and the resulting precipitate was vacuum dried to obtain 24.0 g of compound (a1-1).

20.0 g of compound (a1-1) and 19.0 g of triethylamine were dissolved in 100 g of tetrahydrofuran (hereinafter sometimes referred to as "THF") to obtain a solution. While the solution was stirred at 0°C, 9.3 g of compound (b1-1) was dropwise added to the solution, followed by stirring at 0°C for one hour to obtain a solution of compound (c1-1). Then, the obtained solution of compound (c1-1) was poured into water, followed by extraction three time with ethyl acetate. The resulting organic phase was vacuum dried to obtain 28.0 g of compound (c1-1).

25.0 g of compound (c1-1), 15.0 g of isoamyl nitrite and 12.5 g of a 35 weight% hydrochloric acid aqueous solution were dissolved in 125 g of THF to obtain a solution. The solution was stirred at room temperature for 3 days to obtain a solution of compound (d1-1). Then, the obtained solution of compound (d1-1) was poured into water, followed by extraction three times with ethyl acetate. The resulting organic phase was vacuum dried to obtain 25.0 g of compound (d1-1).

7.5 g of phthalic anhydride, 20.0 g of 1 H, 1 H, 2H, 2H-perfluorooctanol, 8.9 g of 4-dimethylaminopyridine and 7.5 g of triethylamine were dissolved in 200 g of dichloromethane to obtain a solution. The solution was stirred at room temperature for 5 hours to obtain a solution of compound (e1-1). Then, the obtained solution of compound (e1-1) was washed with 1 N hydrochloric acid three times and with water once. The resulting organic phase was vacuum dried to obtain 25.0 g of compound (e1-1).

7.5 g of compound (d1-1) and 3.3 g of diisopropylcarbodiimide were dissolved in 50 g of THF to obtain a solution. While the solution was stirred at 0°C, a solution having 13.5 g of compound (e1-1) dissolved in 13 g of THF was dropwise added thereto. The reaction solution was stirred at 0°C for one hour and then stirred at room temperature for 5 hours to obtain a solution of compound (m1-1). The solvents were removed from the solution by an evaporator, followed by vacuum drying to obtain 18 g of compound (m1-1).

Compound (m1-1) was identified by ¹H-NMR and ¹⁹F-NMR.
NMR spectra of compound (m1-1):
¹H-NMR (300.4 MHz, solvent: CDCl₃, standard: TMS) δ (ppm): 2.11 (s, 3H), 2.54 (m, 2H), 4.60 (t, J=6.3, 2H), 5.88 (m, 1 H), 6.12 (m, 1 H), 6.46 (m, 1 H), 7.19 (m, 5H), 7.86 (m, 7H)
¹⁹F-NMR (282.7 MHz, solvent: CDCl₃, standard: CFCl₃) δ (ppm): -126.6 (m, 2F), -124.0 (m, 4F), -123.3 (m, 2F), -122.3 (m, 2F), -114.0 (m, 2F), -81.2 (m, 3F)

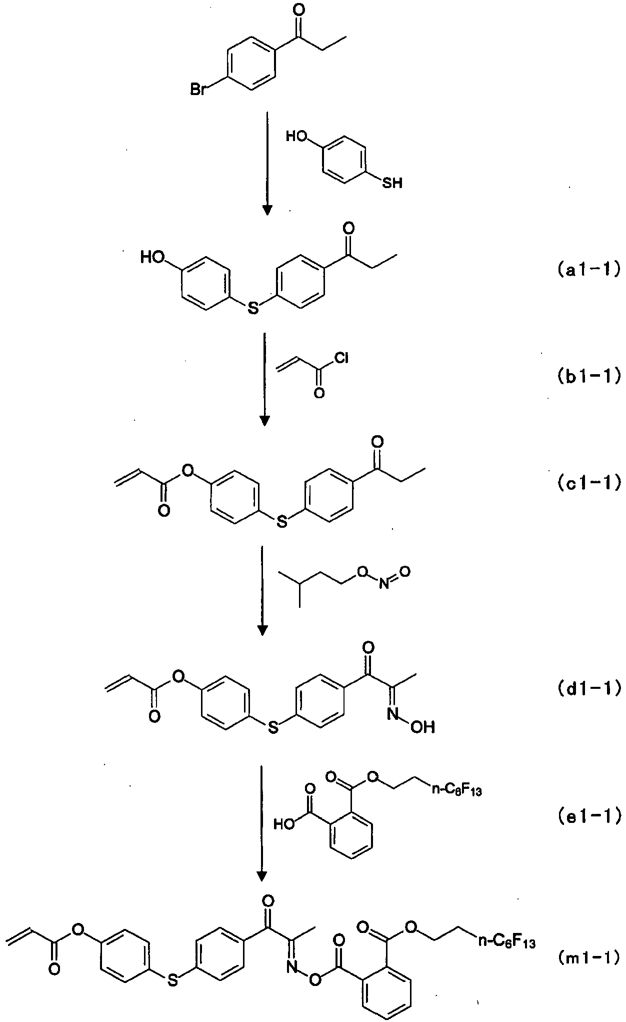

### [Example 2-2]

### (Preparation of compound (m1-2))

A reaction represented by the following formula was carried out. 20.2 g of compound (a1-1) obtained in Example 2-1 and 9.7 g of triethylamine were dissolved in 89 g of THF to obtain a solution. While the solution was stirred at 0°C, 9.0 g of compound (b1-2) was dropwise added to the solution. The reaction solution was stirred at 0°C for one hour and then stirred at room temperature for 3.5 hours to obtain a solution of compound (c1-2). Then, the obtained solution of compound (c1-2) was poured into water, followed by extraction three time with ethyl acetate. The resulting organic phase was vacuum dried to obtain 26.6 g of compound (c1-2).

20.0 g of compound (c1-2), 10.9 g of isoamyl nitrite and 5.0 g of a 35 weight% hydrochloric acid aqueous solution were dissolved in 107 g of THF to obtain a solution. The solution was stirred at room temperature for one day to obtain a solution of compound (d1-2). Then, the obtained solution of compound (d1-2) was poured into water, followed by extraction three times with ethyl acetate. The resulting organic phase was vacuum dried to obtain 14.2 g of compound (d1-2).

10.0 g of compound (d1-2) and 5.5 g of diisopropylcarbodiimide were dissolved in 70 g of THF to obtain a solution. While the solution was stirred at 0°C, a solution having 21.8 g of compound (e1-1) dissolved in 20 g of THF was dropwise added thereto. The reaction solution was stirred at 0°C for one hour and then stirred at room temperature for 20 hours to obtain a suspension of compound (m1-2). The suspension was subjected to filtration, and the solvents were removed from the obtained solution by an evaporator, followed by vacuum drying to obtain 15.0 g of compound (m1-2).

Compound (m1-2) was identified by ¹H-NMR and ¹⁹F-NMR.
NMR spectra of compound (m1-2):
¹H-NMR (300.4 MHz, solvent: CDCl₃, standard: TMS) δ (ppm): 2.11 (s, 3H), 2.28 (s, 3H), 2.54 (m, 2H), 4.60 (t, J=6.3, 2H), 5.88 (s, 1 H), 6.38 (s, 1 H), 7.20 (m, 5H), 7.86 (m, 7H)
¹⁹F-NMR (282.7 MHz, solvent: CDCl₃, standard: CFCl₃) δ (ppm): -126.6 (m, 2F), -124.0 (m, 4F), -123.3 (m, 2F), -122.3 (m, 2F), -114.0 (m, 2F), -81.2 (m, 3F)

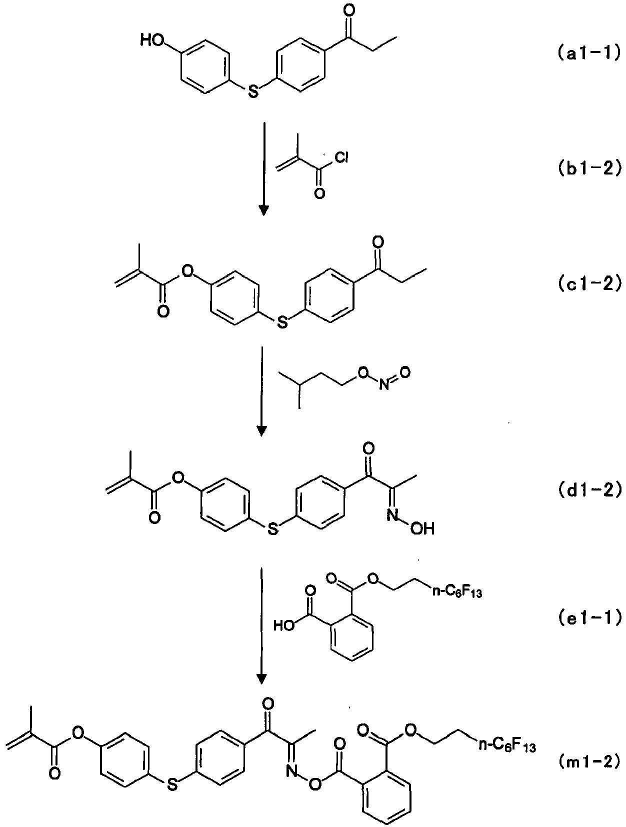

### [Example 2-3]

### (Preparation of compound (m1-3))

3.5 g of succinic anhydride, 13.7 g of 1 H, 1 H, 2H, 2H-perfluorooctanol, 1.1 g of 4-dimethylaminopyridine and 5.4 g of triethylamine were dissolved in 106 g of dichloromethane to obtain a solution. The solution was stirred at room temperature for 17 hours to obtain a solution of compound (e1-2). Then, the obtained solution of compound (e1-2) was washed with 1 N hydrochloric acid three times and with water once. The resulting organic phase was vacuum dried to obtain 15.7 g of compound (e1-2).

A reaction represented by the following formula was carried out. 5.1 g of compound (d1-2) obtained in Example 2-2 and 2.7 g of diisopropylcarbodiimide were dissolved in 30 g of THF to obtain a solution. While the solution was stirred at 0°C, a solution having 9.7 g of compound (e1-2) dissolved in 10 g of THF was dropwise added thereto. The reaction solution was further stirred at 0°C for one hour and then at room temperature for 19 hours to obtain a solution of compound (m1-3). The solvents were removed from the solution by an evaporator, followed by vacuum drying to obtain 9.7 g of compound (m1-3).

Compound (m1-3) was identified by ¹H-NMR and ¹⁹F-NMR.
NMR spectra of compound (m1-3):
¹H-NMR (300.4 MHz, solvent: CDCl₃, standard: TMS) δ (ppm): 2.11 (s, 3H), 2.28 (s, 3H), 2.54 (m, 2H), 2.80 (m, 4H), 4.60 (t, J=6.3, 2H), 5.88 (s, 1 H), 6.38 (s, 1 H), 7.19 (m, 4H), 7.55 (m, 2H), 7.98 (m, 2H)
¹⁹F-NMR (282.7 MHz, solvent: CDCl₃, standard: CFCl₃) δ (ppm): -126.6 (m, 2F), -124.0 (m, 4F), -123.3 (m, 2F), -122.3 (m,2F), -114.0 (m, 2F), -81.2 (m, 3F)

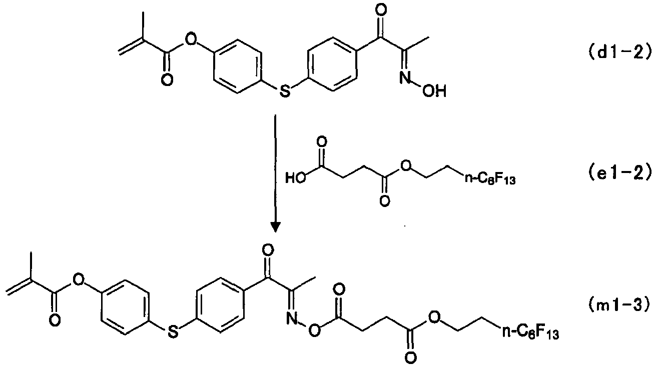

### [Example 2-4]

### (Preparation of compound (m1-4))

5 g of succinic anhydride, 18.6 g of 2,2-difluoro-2(1,1,2,2-tetrafluoro-2-(2-perfluoroethoxy)ethanol, 1.5 g of 4-dimethylaminopyridine and 7.6 g of triethylamine were dissolved in 113 g of dichloromethane to obtain a solution. The solution was stirred at room temperature for 17 hours to obtain a solution of compound (e1-3). Then, the obtained solution of compound (e1-3) was washed with 1 N hydrochloric acid three times and with water once. The resulting organic phase was vacuum dried to obtain 21.4 g of compound (e1-3).

A reaction represented by the following formula was carried out. 12 g of compound (d1-2) obtained in Example 2-2 and 5.6 g of diisopropylcarbodiimide were dissolved in 81 g of THF to obtain a solution. While the solution was stirred at 0°C, a solution having 17.5 g of compound (e1-3) dissolved in 20 g of THF was dropwise added thereto. The reaction solution was further stirred at 0°C for one hour and then at room temperature for 19 hours to obtain a solution of compound (m1-4). The solvents were removed from the solution by an evaporator, followed by vacuum drying to obtain 21 g of compound (m1-4).

Compound (m1-4) was identified by ¹H-NMR and ¹⁹F-NMR.
NMR spectra of compound (m1-4):
¹H-NMR (300.4 MHz, solvent: CDCl₃, standard: TMS) δ (ppm): 2.27 (s, 3H), 2.88 (m, 7H), 4.76 (t, J=9.9, 2H), 5.88 (s, 1 H), 6.34 (s, 1 H), 7.30 (m, 4H), 7.65 (m, 2H), 8.03 (m, 2H)
¹⁹F-NMR (282.7 MHz, solvnet: CDCl₃, standard: CFCl₃) δ (ppm): -88.1 (m, 6F), -86.4 (m, 3F), -76.6 (m, 2F)

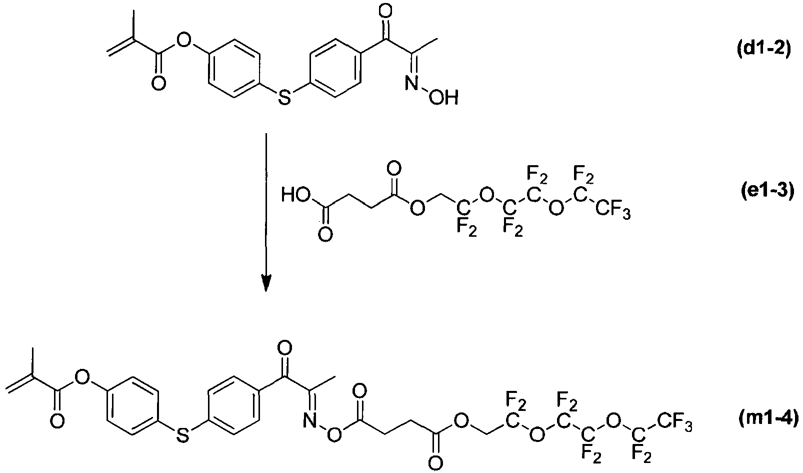

### [Example 3-1]

### (Preparation of polymer (C-1))

In 20.0 g of 2-butanone, 9.8 g of compound (m1-1) and 1.5 g of 2-hydroxyethyl methacrylate were reacted at 50°C for 24 hours in the presence of 0.34 g of n-dodecylmercaptan and 0.05 g of 2,2'-azobis(4-methoxy-2,4-dimethylvaleronitrile) (manufactured by Wako Pure Chemical Industries, Ltd., tradename: V-70). After the reaction mixture was cooled to room temperature (20 to 25°C), 1.7 g of 2-acryloyloxyethyl isocyanate, 0.007 g of dibutyltin dilaurate and 0.084 g of 2,6-di-tert-butyl-p-cresol were added, followed by reaction at 40°C for 24 hours to obtain a 2-butanone solution of polymer (C-1) having the following units (u1-1) and (u2-1). The obtained 2-butanone solution of polymer (C-1) was poured into hexane to precipitate a solid, which was vacuum dried to obtain 10.1 g of powdery polymer (C-1). Of polymer (C-1), the fluorine content was 22.3 weight% and the number average molecular weight (Mn) was 8,000. The results are shown in Table 1.

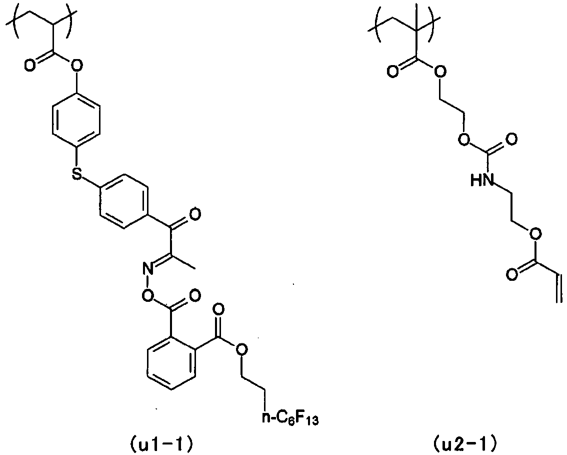

### [Example 3-2]

### (Preparation of polymer (C-2))

In 2.7 g of 2-butanone, 1.0 g of compound (m1-2) and 0.15 g of 2-hydroxyethyl methacrylate were reacted at 50°C for 24 hours in the presence of 0.33 g of n-octadecylmercaptan and 0.005 g of 2,2'-azobis(2,4-dimethylvaleronitrile) (manufactured by Wako Pure Chemical Industries, Ltd., tradename: V-65). After the reaction mixture was cooled to room temperature (20 to 25°C), 0.17 g of 2-acryloyloxyethyl isocyanate, 0.0007 g of dibutyltin dilaurate and 0.008 g of 2,6-di-tert-butyl-p-cresol were added, followed by reaction at 40°C for 24 hours to obtain a 2-butanone solution of polymer (C-2) having the following units (u1-2) and (u2-1). In the same manner as in Example 3-1, 1.18 g of powdery polymer (C-2) was obtained, and the fluorine content and the number average molecular weight (Mn) were measured. The results are shown in Table 1.

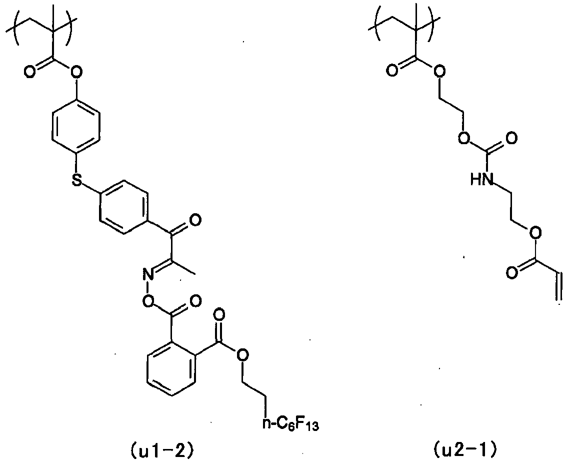

### [Example 3-3]

### (Preparation of polymer (C-3))

In 2.7 g of 2-butanone, 1.0 g of compound (m1-2), 0.12 g of 2-hydroxyethyl methacrylate and 0.02 g of methacrylic acid were reacted at 50°C for 24 hours in the presence of 0.33 g of n-octadecylmercaptan and 0.004 g of 2,2'-azobis(2,4-dimethylvaleronitrile) (manufactured by Wako Pure Chemical Industries, Ltd., tradename: V-65). After the reaction mixture was cooled to room temperature (20 to 25°C), 0.13 g of 2-acryloyloxyethyl isocyanate, 0.0005 g of dibutyltin dilaurate and 0.007 g of 2,6-di-tert-butyl-p-cresol were added, followed by reaction at 40°C for 24 hours to obtain a 2-butanone solution of polymer (C-2) having the following units (u1-2), (u2-1) and (u3-1). In the same manner as in Example 3-1, 0.99 g of powdery polymer (C-3) was obtained, and the fluorine content and the number average molecular weight (Mn) were measured. The results are shown in Table 1.

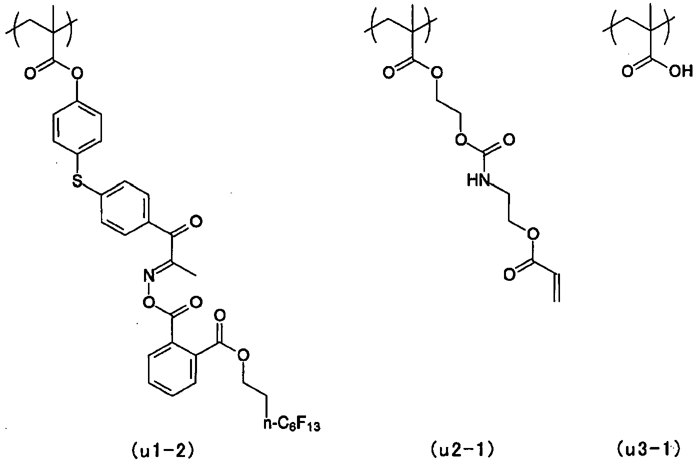

### [Example 3-4]

### (Preparation of polymer (C-4))

In 2.7 g of 2-butanone, 1.0 g of compound (m1-2), 0.12 g of 2-hydroxyethyl methacrylate and 0.07 g of 2-(1H-benzo[d][1,2,3]triazole-1-carboxyamide)ethyl methacrylate were reacted at 50°C for 24 hours in the presence of 0.33 g of n-octadecylmercaptan and 0.004 g of 2,2'-azobis(2,4-dimethylvaleronitrile) (manufactured by Wako Pure Chemical Industries, Ltd., tradename: V-65). After the reaction mixture was cooled to room temperature (20 to 25°C), 0.13 g of 2-acryloyloxyethyl isocyanate, 0.0005 g of dibutyltin dilaurate and 0.007 g of 2,6-di-tert-butyl-p-cresol were added, followed by reaction at 40°C for 24 hours to obtain a 2-butanone solution of polymer (C-4) having the following units (u1-2), (u2-1) and (u3-2). In the same manner as in Example 3-1, 1.06 g of powdery polymer (C-4) was obtained, and the fluorine content and the number average molecular weight (Mn) were measured. The results are shown in Table 1.

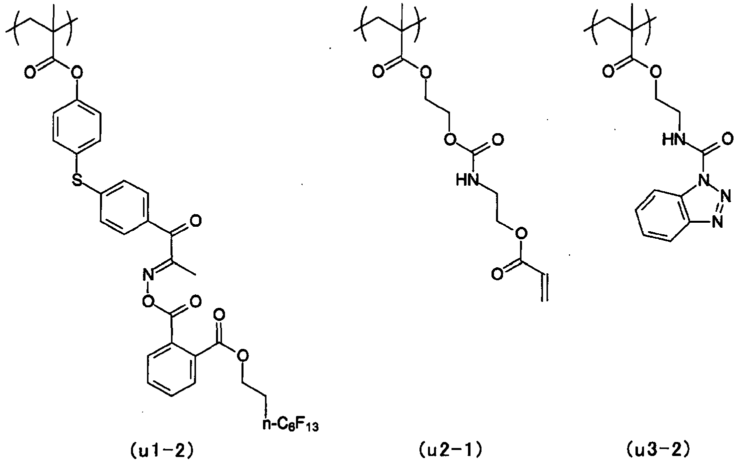

### [Example 3-5]

### (Preparation of polymer (C-5))

In 2.2 g of 2-butanone, 0.8 g of compound (m1-3) and 0.13 g of 2-hydroxyethyl methacrylate were reacted at 50°C for 24 hours in the presence of 0.03 g of n-octadecylmercaptan and 0.004 g of 2,2'-azobis(2,4-dimethylvaleronitrile) (manufactured by Wako Pure Chemical Industries, Ltd., tradename: V-65). After the reaction mixture was cooled to room temperature (20 to 25°C), 0.14 g of 2-acryloyloxyethyl isocyanate, 0.0006 g of dibutyltin dilaurate and 0.007 g of 2,6-di-tert-butyl-p-cresol were added, followed by reaction at 40°C for 24 hours to obtain a 2-butanone solution of polymer (C-5) having the following units (u1-3) and (u2-1). In the same manner as in Example 3-1, 0.89 g of powdery polymer (C-5) was obtained, and the fluorine content and the number average molecular weight (Mn) were measured. The results are shown in Table 1.

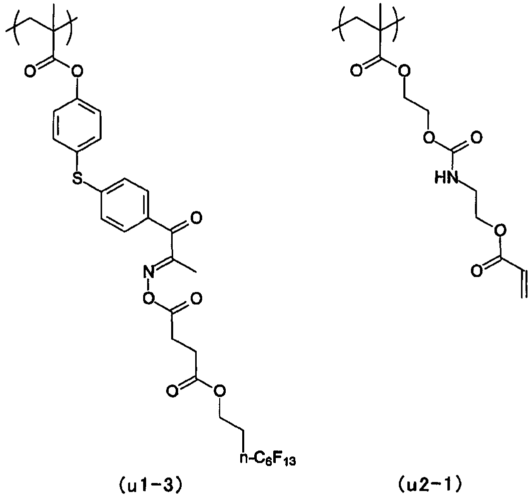

### [Example 3-6]

### (Preparation of polymer (C-6))

In 2.2 g of 2-butanone, 0.8 g of compound (m1-3), 0.08 g of 2-hydroxyethyl methacrylate and 0.11 g of 2-(1 H-benzo[d][1,2,3]triazole-1-carboxyamide)ethyl methacrylate were reacted at 50°C for 24 hours in the presence of 0.02 g of n-octadecylmercaptan and 0.003 g of 2,2'-azobis(2,4-dimethylvaleronitrile) (manufactured by Wako Pure Chemical Industries, Ltd., tradename: V-65). After the reaction mixture was cooled to room temperature (20 to 25°C), 0.08 g of 2-acryloyloxyethyl isocyanate, 0.0003 g of dibutyltin dilaurate and 0.004 g of 2,6-di-tert-butyl-p-cresol were added, followed by reaction at 40°C for 24 hours to obtain a 2-butanone solution of polymer (C-6) having the following units (u1-3), (u2-1) and (u3-2). In the same manner as in Example 3-1, 0.86 g of powdery polymer (C-6) was obtained, and the fluorine content and the number average molecular weight (Mn) were measured. The results are shown in Table 1.

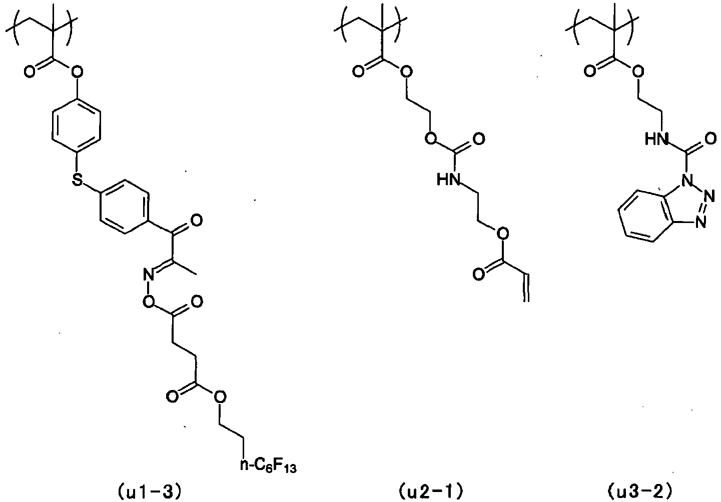

### [Example 3-7]

### (Preparation of polymer (C-7))

0.93 g of powdery polymer (C-7) having the following units (u1-3), (u2-1) and (u3-3) was obtained in the same manner as in Example 3-6 except that 0.11 g of 2-(1H-benzo[d][1,2,3]triazole-1-carboxyamide)ethyl methacrylate was changed to 0.13 g of RUVA-93 (manufactured by Otsuka Chemical Co., Ltd.). The fluorine content and the number average molecular weight (Mn) were measured, and the results are shown in Table 1.

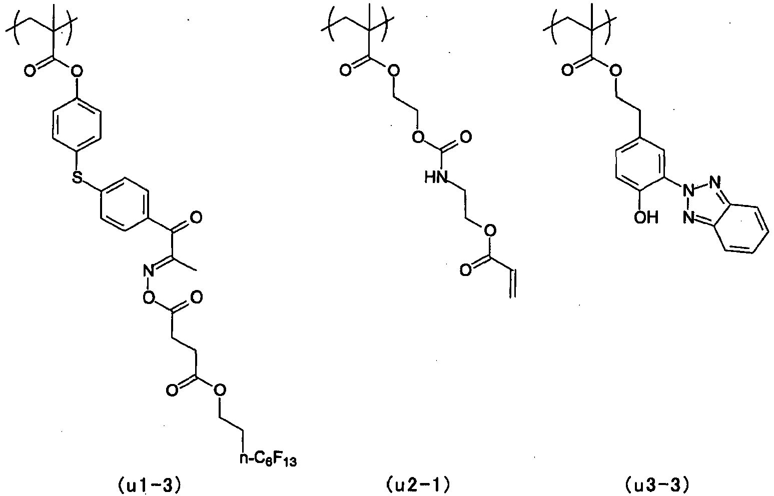

### [Example 3-8]

### (Preparation of polymer (C-8))

0.82 g of powdery polymer (C-8) having the following units (u1-3), (u2-1) and (u3-4) was obtained in the same manner as in Example 3-6 except that 0.11 g of 2-(1H-benzo[d][1,2,3]triazole-1-carboxyamide)ethyl methacrylate was changed to 0.09 g of SA (manufactured by SHIN-NAKAMURA CHEMICAL CO., LTD.). The fluorine content and the number average molecular weight (Mn) were measured, and the results are shown in Table 1.

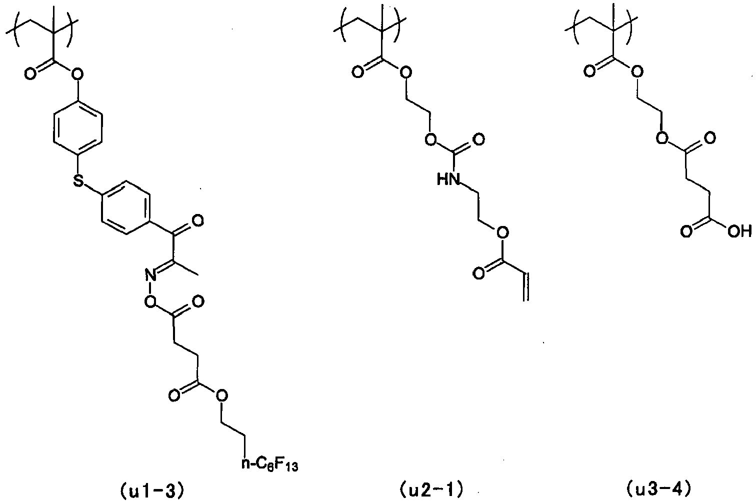

### [Example 3-9]

### (Preparation of polymer (C-9))

0.82 g of powdery polymer (C-9) having the following units (u1-3), (u2-1) and (u3-5) was obtained in the same manner as in Example 3-6 except that 0.11 g of 2-(1H-benzo[d][1,2,3]triazole-1-carboxyamide)ethyl methacrylate was changed to 0.07 g of octyl acrylate. The fluorine content and the number average molecular weight (Mn) were measured, and the results are shown in Table 1.

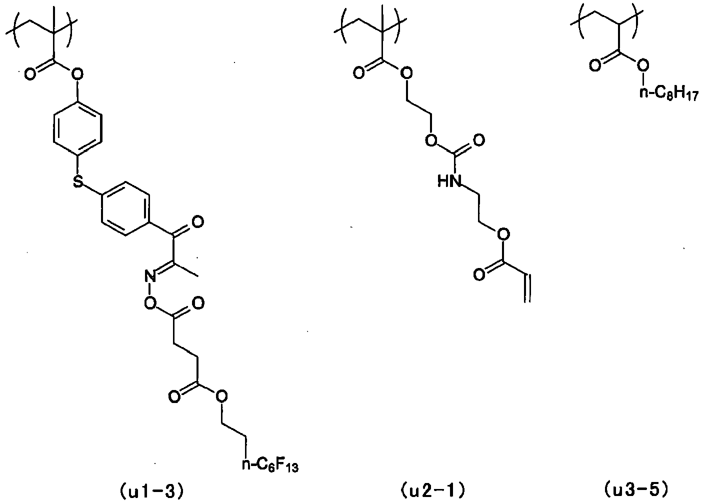

### [Example 3-10]

### (Preparation of polymer (C-10))

0.95 g of powdery polymer (C-10) having the following units (u1-3), (u2-1) and (u3-6) was obtained in the same manner as in Example 3-6 except that 0.11 g of 2-(1H-benzo[d][1,2,3]triazole-1-carboxyamide)ethyl methacrylate was changed to 0.096 g of decyl acrylate. The fluorine content and the number average molecular weight (Mn) were measured, and the results are shown in Table 1.

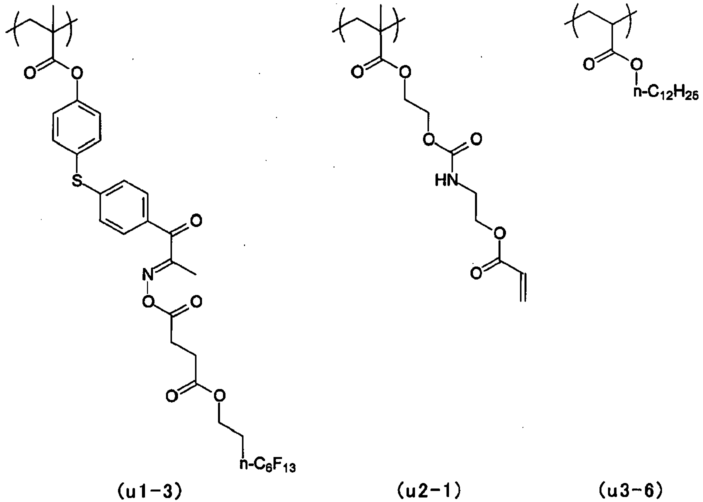

**[Table 1]**

| Ex. | | | 3-1 | 3-2 | 3-3 | 3-4 | 3-5 | 3-6 | 3-7 | 3-8 | 3-9 | 3-10 |
|---|---|---|---|---|---|---|---|---|---|---|---|---|
| Polymer (C) | | | (C-1) | (C-2) | (C-3) | (C-4) | (C-5) | (C-6) | (C-7) | (C-8) | (C-9) | (C-10) |
| Proportion of units in polymer (C) (weight%) | Units (u1) | Units (u1-1) | 75 | - | - | - | - | - | - | - | - | - |
| | | Units (u1-2) | - | 76 | 78 | 76 | - | - | - | - | - | - |
| | | Units (u1-3) | - | - | - | - | 75 | 75 | 73 | 76 | 77 | 76 |
| | Units (u2) | Units (u2-1) | 25 | 24 | 20 | 19 | 25 | 15 | 15 | 15 | 16 | 15 |
| | Units (u3) | Units (u3-1) | - | - | 2 | - | - | - | - | - | - | - |
| | | Units (u3-2) | - | - | - | 5 | - | 10 | - | - | - | - |
| | | Units (u3-3) | - | - | - | - | - | - | 12 | - | - | - |
| | | Units (u3-4) | - | - | - | - | - | - | - | 9 | - | - |
| | | Units (u3-5) | - | - | - | - | - | - | - | - | 7 | - |
| | | Units (u3-6) | - | - | - | - | - | - | - | - | - | 9 |
| Fluorine content (weight%) | | | 22.3 | 22.0 | 22.8 | 22.0 | 23.0 | 23.0 | 22.6 | 23.4 | 23.8 | 23.3 |
| Number average molecular weight (Mn) | | | 8,000 | 8,000 | 7,000 | 8,000 | 8,000 | 7,000 | 7,000 | 7,000 | 8,000 | 7,000 |

### [Example 3-11]

### (Preparation of polymer (C-11))

In 2.4 g of 2-butanone, 0.9 g of compound (m1-4) and 0.10 g of 2-hydroxyethyl methacrylate were reacted at 50°C for 24 hours in the presence of 0.03 g of n-octadecylmercaptan and 0.004 g of 2,2'-azobis(2,4-dimethylvaleronitrile) (manufactured by Wako Pure Chemical Industries, Ltd., tradename: V-65). After the reaction mixture was cooled to room temperature (20 to 25°C), 0.11 g of 2-acryloyloxyethyl isocyanate, 0.0004 g of dibutyltin dilaurate and 0.006 g of 2,6-di-tert-butyl-p-cresol were added, followed by reaction at 40°C for 24 hours to obtain a 2-butanone solution of polymer (C-11) having the following units (u1-4) and (u2-1). In the same manner as in Example 3-1, 0.82 g of powdery polymer (C-11) was obtained, and the fluorine content and the number average molecular weight (Mn) were measured. The results are shown in Table 2.

### [Example 3-12]

### (Preparation of polymer (C-12))

In 2.1 g of 2-butanone, 0.7 g of compound (m1-4) and 0.18 g of 2-hydroxyethyl methacrylate were reacted at 50°C for 24 hours in the presence of 0.03 g of n-octadecylmercaptan and 0.004 g of V-65. After the reaction mixture was cooled to room temperature (20 to 25°C), 0.19 g of 2-acryloyloxyethyl isocyanate, 0.0008 g of dibutyltin dilaurate and 0.010 g of 2,6-di-tert-butyl-p-cresol were added, followed by reaction at 40°C for 24 hours to obtain a 2-butanone solution of polymer (C-12) having the following units (u1-4) and (u2-1). In the same manner as in Example 3-1, 0.93 g of powdery polymer (C-12) was obtained, and the fluorine content and the number average molecular weight (Mn) were measured. The results are shown in Table 2.

### [Example 3-13]

### (Preparation of polymer (C-13))

In 2.3 g of 2-butanone, 0.9 g of compound (m1-4), 0.03 g of 2-hydroxyethyl methacrylate and 0.06 g of styrene were reacted at 50°C for 24 hours in the presence of 0.03 g of n-octadecylmercaptan and 0.004 g of V-65. After the reaction mixture was cooled to room temperature (20 to 25°C), 0.03 g of 2-acryloyloxyethyl isocyanate, 0.0001 g of dibutyltin dilaurate and 0.001 g of 2,6-di-tert-butyl-p-cresol were added, followed by reaction at 40°C for 24 hours to obtain a 2-butanone solution of polymer (C-13) having the following units (u1-4), (u2-1) and (u3-7). In the same manner as in Example 3-1, 0.62 g of powdery polymer (C-13) was obtained, and the fluorine content and the number average molecular weight (Mn) were measured. The results are shown in Table 2.

### [Example 3-14]

### (Preparation of polymer (C-14))

In 2.3 g of 2-butanone, 0.8 g of compound (m1-4), 0.03 g of 2-hydroxyethyl methacrylate and 0.14 g of styrene were reacted at 50°C for 24 hours in the presence of 0.04 g of n-octadecylmercaptan and 0.005 g of V-65. After the reaction mixture was cooled to room temperature (20 to 25°C), 0.04 g of 2-acryloyloxyethyl isocyanate, 0.0001 g of dibutyltin dilaurate and 0.002 g of 2,6-di-tert-butyl-p-cresol were added, followed by reaction at 40°C for 24 hours to obtain a 2-butanone solution of polymer (C-14) having the following units (u1-4), (u2-1) and (u3-7). In the same manner as in Example 3-1, 0.57 g of powdery polymer (C-14) was obtained, and the fluorine content and the number average molecular weight (Mn) were measured. The results are shown in Table 2.

### [Example 3-15]

### (Preparation of polymer (C-15))

0.67 g of powdery polymer (C-15) having the after-mentioned units (u1-4), (u2-1) and (u3-8) was obtained in the same manner as in Example 3-13 except that 0.06 g of styrene was changed to 0.10 g of hexyl methacrylate. The fluorine content and the number average molecular weight (Mn) were measured, and the results are shown in Table 2.

### [Example 3-16]

### (Preparation of polymer (C-16))

0.57 g of powdery polymer (C-16) having the after-mentioned units (u1-4), (u2-1) and (u3-8) was obtained in the same manner as in Example 3-14 except that 0.14 g of styrene was changed to 0.22 g of hexyl methacrylate. The fluorine content and the number average molecular weight (Mn) were measured, and the results are shown in Table 2.

### [Example 3-17]

### (Preparation of polymer (C-17))

0.70 g of powdery polymer (C-17) having the after-mentioned units (u1-4), (u2-1) and (u3-9) was obtained in the same manner as in Example 3-13 except that 0.06 g of styrene was changed to 0.15 g of dodecyl methacrylate. The fluorine content and the number average molecular weight (Mn) were measured, and the results are shown in Table 2.

### [Example 3-18]

### (Preparation of polymer (C-18))

0.11 g of powdery polymer (C-18) having the after-mentioned units (u1-4), (u2-1) and (u3-9) was obtained in the same manner as in Example 3-14 except that 0.14 g of styrene was changed to 0.33 g of dodecyl methacrylate. The fluorine content and the number average molecular weight (Mn) were measured, and the results are shown in Table 2.

### [Example 3-19]

### (Preparation of polymer (C-19))

0.89 g of powdery polymer (C-19) having the after-mentioned units (u1-4) and (u2-1) and the above units (u3-3) was obtained in the same manner as in Example 3-13 except that 0.06 g of styrene was changed to 0.19 g of RUVA-93. The fluorine content and the number average molecular weight (Mn) were measured, and the results are shown in Table 2.

### [Example 3-20]

### (Preparation of polymer (C-20))

1.11 g of powdery polymer (C-20) having the after-mentioned units (u1-4) and (u2-1) and the above units (u3-3) was obtained in the same manner as in Example 3-14 except that 0.14 g of styrene was changed to 0.42 g of RUVA-93. The fluorine content and the number average molecular weight (Mn) were measured, and the results are shown in Table 2.

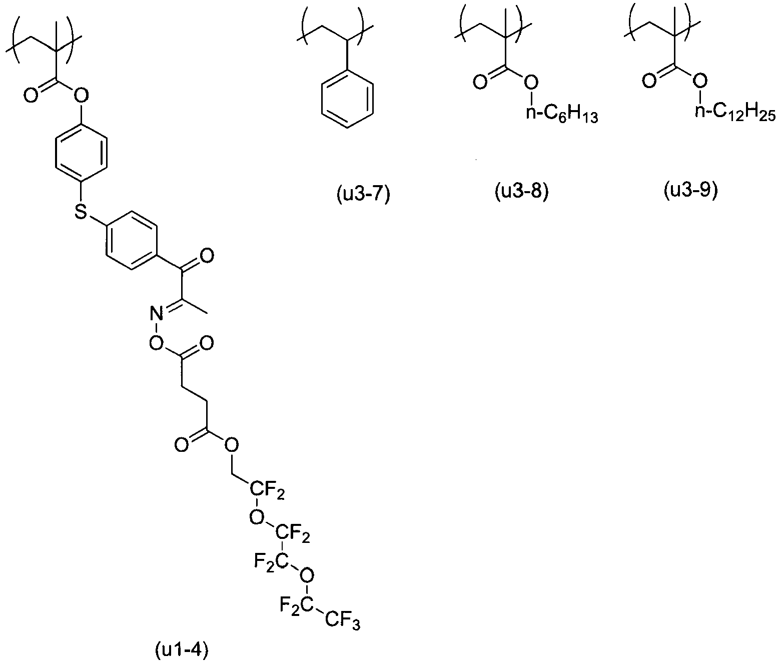

**[Table 2]**

| Ex. | | | 3-11 | 3-12 | 3-13 | 3-14 | 3-15 | 3-16 | 3-17 | 3-18 | 3-19 | 3-20 |
|---|---|---|---|---|---|---|---|---|---|---|---|---|
| Polymer (C) | | | (C-11) | (C-12) | (C-13) | (C-14) | (C-15) | (C-16) | (C-17) | (C-18) | (C-19) | (C-20) |
| Proportion of units in polymer (C) (weight%) | Units (u1) | Units (u1-4) | 81 | 65 | 89 | 80 | 86 | 73 | 82 | 67 | 79 | 62 |
| | Units (u2) | Units (u2-1) | 19 | 35 | 5 | 7 | 5 | 7 | 5 | 6 | 5 | 6 |
| | Units (u3) | Units (u3-3) | - | - | - | - | - | - | - | - | 17 | 33 |
| | | Units (u3-7) | - | - | 6 | 14 | - | - | - | - | - | - |
| | | Units (u3-8) | - | - | - | - | 10 | 20 | - | - | - | - |
| | | Units (u3-9) | - | - | - | - | - | - | 14 | 28 | - | - |
| Fluorine content (weight%) | | | 22 | 17.8 | 24.1 | 21.6 | 23.2 | 19.9 | 22.2 | 18.1 | 21.4 | 16.8 |
| Number average molecular weight (Mn) | | | 16,000 | 13,000 | 12,000 | 9,000 | 17,000 | 14,000 | 19,000 | 17,000 | 19,000 | 13,000 |

### [Example 4-1]

### (Preparation of coating composition)

1.2 g of prepolymer (A1-1), 0.8 g of dipentaerythritol hexaacrylate (number average molecular weight (Mn): 578) as the compound (B), 0.01 g of polymer (C-1) prepared in Example 3-1 and 0.2 g of benzoyl peroxide as the thermal polymerization initiator (D1) were dissolved in 8.0 g of PGMEA to prepare coating composition (1).

### [Examples 4-2 to 4-20]

### (Preparation of coating composition)

Coating compositions (2) to (20) were prepared in the same manner as in Example 4-1 except that ditrimethylolpropane hexaacrylate was used as the compound (B) and polymers (C-2) to (2-20) were used.

### [Examples 5-1 to 5-20]

### (Production of article having cured film)

The coating composition prepared in each of Examples 4-1 to 4-20 was applied to a glass substrate (manufactured by Corning Incorporated, 50 mm × 50 mm × 0.725 mm in thickness) by spin coating at 1,000 revolutions per minute for 30 seconds to obtain a coating film. Then, the glass substrate provided with the coating film was heated by a hot plate at 60°C for 90 seconds to form a dry film having a thickness of 1 µm.

The surface of the dry film was partially irradiated with ultraviolet light (i-line: 365 nm) by means of a mask pattern. Irradiation with ultraviolet light was carried out using MA-8, tradename, manufactured by SUSS under irradiation conditions of 1 J/cm². By this apparatus under these conditions, ultraviolet light having a wavelength of at most 350 nm is not applied.

Then, the glass substrate provided with the dry film having a pattern formed thereon was heated by an oven at 150°C for 30 minutes to cure the dry film having a pattern formed thereon.

The PGMEA contact angle of the portion irradiated with ultraviolet light and the PGMEA contact angle of the portion not irradiated with ultraviolet light were measured. The results are shown in Tables 3 and 4.

**[Table 3]**

| Ex. | 5-1 | 5-2 | 5-3 | 5-4 | 5-5 | 5-6 | 5-7 | 5-8 | 5-9 | 5-10 |
|---|---|---|---|---|---|---|---|---|---|---|
| Coating composition used | (1) | (2) | (3) | (4) | (5) | (6) | (7) | (8) | (9) | (10) |
| Polymer (C) used | (C-1) | (C-2) | (C-3) | (C-4) | (C-5) | (C-6) | (C-7) | (C-8) | (C-9) | (C-10) |
| PGMEA contact angle (°) of portion irradiated with ultraviolet light | 10≧ | 10≧ | 10≧ | 10≧ | 10≧ | 10≧ | 10≧ | 10≧ | 10≧ | 10≧ |
| PGMEA contact angle (°) of portion not irradiated with ultraviolet light | 35 | 40 | 42 | 43 | 51 | 51 | 48 | 51 | 51 | 52 |

**[Table 4]**

| Ex. | 5-11 | 5-12 | 5-13 | 5-14 | 5-15 | 5-16 | 5-17 | 5-18 | 5-19 | 5-20 |
|---|---|---|---|---|---|---|---|---|---|---|
| Coating composition used | (11) | (12) | (13) | (14) | (15) | (16) | (17) | (18) | (19) | (20) |
| Polymer (C) used | (C-11) | (C-12) | (C-13) | (C-14) | (C-15) | (C-16) | (C-17) | (C-18) | C-19) | (C-20) |
| PGMEA contact angle (°) of portion irradiated with ultraviolet light | 10≧ | 10≧ | 10≧ | 10≧ | 10≧ | 10≧ | 10≧ | 10≧ | 10≧ | 10≧ |
| PGMEA contact angle (°) of portion not irradiated with ultraviolet light | 58 | 52 | 58 | 55 | 55 | 48 | 48 | 35 | 58 | 52 |

The polymer (C) of the present invention was decomposed in its molecule by irradiation with ultraviolet light having a wavelength of at least 300 nm, and a decomposition residue containing a fluoroalkyl group could leave.

Further, by using a curable composition containing the polymer (C) of the present invention, an article having a pattern of a liquid-philic region and a liquid repellent region could be produced.

Particularly when a curable composition containing a polymer (C) having units based on a compound (m1) wherein R² is -(CH₂)_{w3}COO(CH₂)_{w4}- was used, the PGMEA contact angle of the portion not irradiated with ultraviolet light was large, and the difference in the PGMEA contact angle between the liquid-philic region and the liquid repellent region (Examples 5-5 to 5-20) was remarkable. That is, a pattern having a greater difference in the liquid repellency between the liquid-philic region and the liquid repellent region could be formed.

### INDUSTRIAL APPLICABILITY

The application of a cured film made of the curable composition of the present invention may, for example, be an electrical insulation film, a chemical or physical protective film, a non-adhesive film, etc. in various electronic devices (such as a semiconductor device). Specifically, application to an interlayer insulation film for a flexible device, a protective film for a flexible device, a gate insulation film for an organic thin-film transistor, a gate insulation film for an oxide thin-film transistor, a capacitor insulation film, a gate insulation film of a memory transistor, a passivation of a semiconductor, a protective film of a semiconductor device, an interlayer insulation film of multilayer interconnection for high density mounting, an insulating layer of an organic electroluminescence device, an insulation film for re-wiring, a cover coating of a flexible copper-clad plate, a solder resist film, a liquid crystal alignment film, a protective film for a color filter, a resin post for e.g. a semiconductor device, and partition walls for e.g. a color filter, etc. may be mentioned.

The entire disclosures of Japanese Patent Application No. 2012-018086 filed on January 31, 2012 and Japanese Patent Application No. 2012-069928 filed on March 26, 2012 including specifications, claims, drawings and summaries are incorporated herein by reference in their entireties.

### REFERENCE SYMBOLS

- 10:: Organic thin-film transistor
- 12:: Substrate
- 14:: Gate electrode
- 15:: Dry film
- 15b:: Liquid repellent region
- 15c:: Internal region
- 16:: Gate insulation film
- 16a:: Liquid-philic region
- 16b:: Liquid repellent region
- 16c:: Internal region
- 18:: Source electrode
- 20:: Drain electrode
- 22:: Organic semiconductor layer

## Claims

1. A compound represented by the following formula (m1): 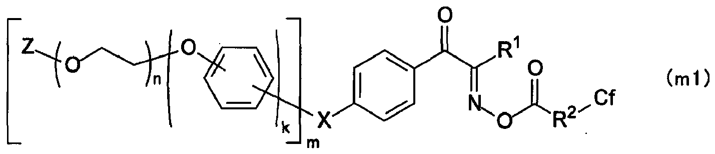
wherein R¹ is a hydrogen atom, a C₁₋₆ alkyl group or a phenyl group,
R² is a single bond or a bivalent organic group having no fluorine atoms,
Cf is a C₁₋₂₀ fluoroalkyl group or a C₂₋₂₀ fluoroalkyl group having an etheric oxygen atom between carbon atoms,
X is an oxygen atom, a sulfur atom, a nitrogen atom or NH,
m is 1 when X is an oxygen atom, a sulfur atom or NH, or 2 when X is a nitrogen atom,
n is an integer of from 0 to 4,
k is 0 or 1,
Z is R⁴R⁵C=CR³-CO-, and
each of R³, R⁴ and R⁵ which are independent of one another, is a hydrogen atom or a methyl group.

2. The compound according to Claim 1, wherein R² is a single bond, -(CH₂)_{w0}-(wherein w0 is an integer of from 1 to 6), -C₆H₄-, -C₆H₄O(CH₂)_{w1}- (wherein w1 in an integer of from 0 to 10), -C₆H₄COO(CH₂)_{w2}- (wherein w2 is an integer of from 0 to 10), -(CH₂)_{w3}COO(CH₂)_{w4}- (wherein w3 is an integer of from 1 to 10, and w4 is an integer of from 0 to 10), -CH₂O(CH₂)_{w5}- (wherein w5 is an integer of from 0 to 10) or -CH (CH₃)O(CH₂)_{w6}- (wherein w6 is an integer of from 0 to 10).

3. A polymer having units (u1) based on the compound as defined in Claim 1 or 2.

4. The polymer according to Claim 3, which further has units (u2) having a crosslinkable functional group and having no Cf group.

5. A curable composition comprising the polymer as defined in Claim 3 or 4.

6. The curable composition according to Claim 5, which further contains a radical polymerization initiator (D).

7. The curable composition according to Claim 5 or 6, which further contains a fluorinated polyarylene prepolymer (A) having a crosslinkable functional group.

8. The curable composition according to Claim 7, which further contains a compound (B) having a number average molecular weight of from 140 to 5,000, having a crosslinkable functional group and having no fluorine atoms.

9. The curable composition according to any one of Claims 5 to 8, wherein the fluorinated polyarylene prepolymer (A) is a prepolymer having a crosslinkable functional group and an ether bond, obtained by subjecting either one or both of a compound (x1) having a crosslinkable functional group and a phenolic hydroxy group and a compound (x2) having a crosslinkable functional group and a fluorinated aromatic ring, a compound (y) represented by the following formula (y), and a compound (z) having at least three phenolic hydroxy groups, to a condensation reaction in the presence of a hydrogen halide-removing agent: 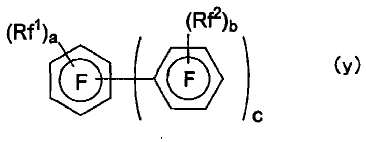 wherein c is an integer of from 0 to 3, a is an integer of from 0 to 3, b is an integer of from 0 to 3, Rf¹ is a fluoroalkyl group having at most 8 carbon atoms, provided that when there are a plurality of Rf¹'s, the plurality of Rf¹'s may be the same or different, Rf² is a fluoroalkyl group having at most 8 carbon atoms, provided that when there are a plurality of Rf²'s, the plurality of Rf²'s may be the same or different, and F in the aromatic ring represents that hydrogen atoms of the aromatic ring are all substituted by fluorine atoms.

10. A coating composition comprising the curable composition as defined in any one of Claims 5 to 9 and a solvent (E).

11. An article comprising a substrate, and a cured film obtained by curing the curable composition as defined in any one of Claims 5 to 9 on the surface of the substrate.

12. An article having a pattern of a liquid-philic region and a liquid repellent region on the surface of a cured film, wherein the liquid repellent region comprises a cured film obtained by curing the curable composition as defined in any one of Claims 5 to 9.

13. The article according to Claim 12, which further has at least one member selected from the group consisting of an electrode, a semiconductor layer, a conductor layer, a transistor material and a resin layer, formed on the surface of the liquid-philic region.

14. A process for producing an article having a pattern of a liquid-philic region and a liquid repellent region, which comprises forming a film of the curable composition as defined in any one of Claims 5 to 9 on the surface of a substrate, curing the film of the curable composition to form a cured film, and partially irradiating the surface of the cured film with ultraviolet light to form a pattern of a liquid-philic region and a liquid repellent region on the surface of the cured film.

15. A process for producing an article having a pattern of a liquid-philic region and a liquid repellent region, which comprises forming a film of the curable composition as defined in any one of Claims 5 to 9 on the surface of a substrate, partially irradiating the surface of the film of the curable composition with ultraviolet light to form a pattern of a liquid-philic region and a liquid repellent region on the surface of the film, and curing the curable composition.
